(19) Europäisches Patentamt / European Patent Office / Office européen des brevets

(11) **EP 4 286 382 A1**

(12) **EUROPEAN PATENT APPLICATION**
published in accordance with Art. 153(4) EPC


(43) Date of publication:
**06.12.2023 Bulletin 2023/49**

(21) Application number: **22745281.0**

(22) Date of filing: **26.01.2022**

(51) International Patent Classification (IPC):
***C07D 471/04** (2006.01)* ***A61P 35/00** (2006.01)*
***A61K 31/437** (2006.01)*

(52) Cooperative Patent Classification (CPC):
**A61K 31/437; A61P 35/00; C07D 471/04**

(86) International application number:
**PCT/CN2022/074076**

(87) International publication number:
**WO 2022/161408 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.01.2021 CN 202110106007**



(71) Applicant: **CGeneTech (Suzhou, China) Co., Ltd.**
**Suzhou, Jiangsu 215123 (CN)**

(72) Inventors:
- **CHEN, Zhengxia**
  **Shanghai 200131 (CN)**
- **DAI, Meibi**
  **Shanghai 200131 (CN)**
- **ZHANG, Yang**
  **Shanghai 200131 (CN)**
- **CHEN, Shuhui**
  **Shanghai 200131 (CN)**

(74) Representative: **Haseltine Lake Kempner LLP**
**One Portwall Square**
**Portwall Lane**
**Bristol BS1 6BH (GB)**


(54) **CRYSTAL FORM OF METHYLPYRAZOLE-SUBSTITUTED PYRIDOIMIDAZOLE COMPOUND AND PREPARATION METHOD THEREFOR**


(57) A crystal form of a methylpyrazole-substituted pyridoimidazole compound (II) and a preparation method therefor.

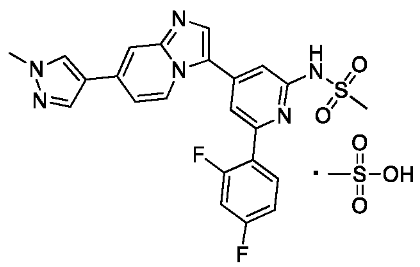

(II)


EP 4 286 382 A1

## Description

The present application claims the following priority: CN202110106007.1, filed on January 26, 2021.

TECHNICAL FIELD

The present disclosure relates to a crystal form of a methylpyrazole-substituted pyridoimidazole compound and a preparation method therefor.

BACKGROUND

Fibroblast growth factor receptor (FGFR) is a class of receptor proteins that can specifically bind to fibroblast growth factor (FGF). The FGFRs family includes the following types: FGFR1b, FGFR1c, FGFR2b, FGFR2c, FGFR3b, FGFR3c and FGFR4. Fibroblast growth factor receptor (FGFR) is a class of bioactive substances that can transmit biological signals, regulate cell growth and participate in tissue repair. It has been clinically found that high expression, mutation or fusion and other abnormalities of FGFR can cause tumor occurrence and development, for example in liver cancer, bladder cancer, lung cancer, breast cancer and other diseases. FGFR binds to the ligand FGF, resulting in the autophosphorylation of multiple intracellular tyrosine residues for downstream signal transduction, including MEK/MAPK, PLCy/PKC, PI3K/AKT, STATS, etc. Therefore, FGFR is considered to be an important antitumor target.

The VEGFR family includes three specific tyrosine kinase receptors, VEGFR-1, VEGFR-2 (KDR) and VEGFR-3. VEGFR-2 is an important regulator of VEGF signal transduction that causes endothelial cell proliferation, increases vascular permeability effect and promotes angiogenesis, and the affinity of VEGFR-2 and VEGF are greater than that of VEGFR-1. Studies have shown that only VEGFR-2 is expressed in endothelial cells and that activation of VEGFR-2 can efficiently stimulate angiogenesis. Therefore, VEGFR-2 is a main target for the development of anti-angiogenesis drugs.

VEGFR and FGFR pathways work together to complete the activation and generation of endothelial cells in angiogenesis, and sometimes VEGF requires the presence of FGF to exert its pro-angiogenic effect. The synergistic effect of FGFR and VEGFR pathways can also inhibit tumor immune escape and improve tumor suppression effect.

CONTENT OF THE PRESENT INVENTION

The present disclosure provides a compound represented by formula (II),

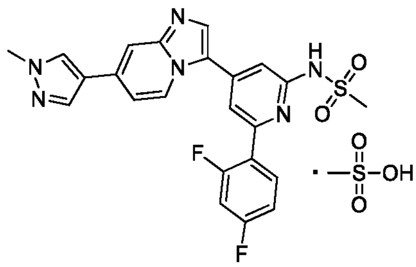

(II)

The present disclosure provides a crystal form A of a compound represented by formula (II), wherein the crystal form A has an X-ray powder diffraction pattern comprising characteristic diffraction peaks at the following 2θ angles: 7.65±0.20°, 17.70±0.20°, 24.02±0.20°,

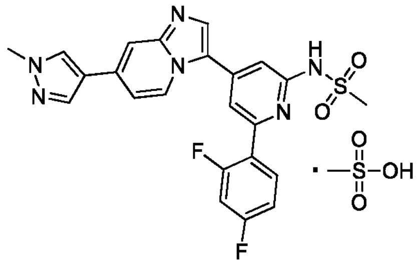

(II)

In some embodiments of the present disclosure, the X-ray powder diffraction pattern of the crystal form A has characteristic diffraction peaks at the following 2θ angles: 7.65±0.20°, 16.84±0.20°, 17.70±0.20°, 20.10±0.20°, 20.91±0.20°, 24.02±0.20°.

In some embodiments of the present disclosure, the X-ray powder diffraction pattern of the crystal form A has characteristic diffraction peaks at the following 2θ angles: 7.65±0.20°, 16.84±0.20°, 17.70±0.20°, 20.10±0.20°, 20.91±0.20°, 24.02±0.20°, 24.98±0.20°, 26.60±0.20°.

In some embodiments of the present disclosure, the X-ray powder diffraction pattern of the crystal form A has characteristic diffraction peaks at the following 2θ angles: 7.65±0.20°, 17.70±0.20°, and/or 24.02±0.20°, and/or 16.84±0.20°, and/or 20.10±0.20°, and/or 20.91±0.20°, and/or 24.98±0.20°, and/or 26.60±0.20°, and/or 12.71±0.20°, and/or 28.08±0.20°.

In some embodiments of the present disclosure, the X-ray powder diffraction pattern of the crystal form A has characteristic diffraction peaks at the following 2θ angles: 7.649°, 12.713°, 16.841°, 17.695°, 20.100°, 20.912°, 24.018°, 24.976°, 26.599°, 28.076°.

In some embodiments of the present disclosure, the XRPD pattern of the crystal form A is shown in FIG. 1.

In some embodiments of the present disclosure, the XRPD pattern analysis data of the crystal form A is shown in Table 1:

**Table 1: XRPD analysis data of the crystal form A of the compound represented by formula (II)**

| No. | 2θ angle (°) | Interplanar spacing (Å) | Intensity (count) | Relative intensity (%) | No. | 2θ angle (°) | Interplanar spacing (Å) | Intensity (count) | Relative intensity (%) |
|---|---|---|---|---|---|---|---|---|---|
| 1 | 7.649 | 11.548 | 258.0 | 100.0 | 6 | 20.912 | 4.245 | 80.6 | 31.3 |
| 2 | 12.713 | 6.957 | 50.8 | 19.7 | 7 | 24.018 | 3.702 | 242.0 | 93.8 |
| 3 | 16.841 | 5.260 | 87.5 | 34.0 | 8 | 24.976 | 3.562 | 79.0 | 30.7 |
| 4 | 17.695 | 5.008 | 245.0 | 95.2 | 9 | 26.599 | 3.348 | 70.4 | 27.3 |
| 5 | 20.100 | 4.414 | 86.3 | 33.5 | 10 | 28.076 | 3.176 | 56.9 | 22.1 |

In some embodiments of the present disclosure, the differential scanning calorimetry curve of the crystal form A has an endothermic peak with a peak value at 283.9±3.0°C.

In some embodiments of the present disclosure, the DSC pattern of the crystal form A is shown in FIG. 2.

In some embodiments of the present disclosure, the thermogravimetric analysis curve of the crystal form A has a weight loss of 0.955% at 200.0±3.0°C.

In some embodiments of the present disclosure, the TGA pattern of the crystal form A is shown in FIG. 3.

The present disclosure provides a crystal form B of a compound represented by formula (II), wherein the crystal form B has an X-ray powder diffraction pattern comprising characteristic diffraction peaks at the following 2θ angles: 6.75±0.20°, 9.94±0.20°, 23.94±0.20°,

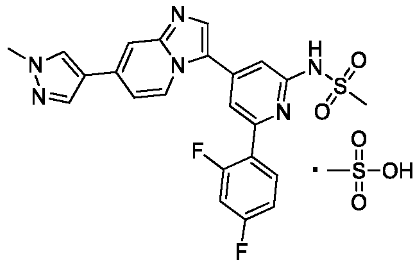

(II)

In some embodiments of the present disclosure, the X-ray powder diffraction pattern of the crystal form B has characteristic diffraction peaks at the following 2θ angles: 6.75±0.20°, 9.94±0.20°, 11.70±0.20°, 17.52±0.20°, 20.36±0.20°, 23.94±0.20°.

In some embodiments of the present disclosure, the X-ray powder diffraction pattern of the crystal form B has characteristic diffraction peaks at the following 2θ angles: 6.75±0.20°, 9.94±0.20°, 11.70±0.20°, 14.38±0.20°, 17.52±0.20°, 18.95±0.20°, 20.36±0.20°, 23.94±0.20°.

In some embodiments of the present disclosure, the X-ray powder diffraction pattern of the crystal form B has characteristic diffraction peaks at the following 2θ angles: 6.75°, 9.94°, 11.70°, 13.62°, 14.38°, 15.47°, 17.52°, 18.95°, 20.36°, 23.94°, 25.34°, 25.46°, 26.93°, 28.79°.

In some embodiments of the present disclosure, the XRPD pattern of the crystal form B is shown in FIG. 4.

In some embodiments of the present disclosure, the XRPD pattern analysis data of the crystal form B is shown in Table 2:

**Table 2: XRPD analysis data of the crystal form B of the compound represented by formula (II)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.75 | 13.10 | 100.00 | 8 | 18.95 | 4.68 | 6.44 |
| 2 | 9.94 | 8.90 | 7.74 | 9 | 20.36 | 4.36 | 7.62 |
| 3 | 11.70 | 7.57 | 6.04 | 10 | 23.94 | 3.72 | 10.37 |
| 4 | 13.62 | 6.50 | 4.18 | 11 | 25.34 | 3.51 | 8.35 |
| 5 | 14.38 | 6.16 | 5.73 | 12 | 25.46 | 3.50 | 8.23 |
| 6 | 15.47 | 5.73 | 4.07 | 13 | 26.93 | 3.31 | 1.85 |
| 7 | 17.52 | 5.06 | 6.83 | 14 | 28.79 | 3.10 | 1.96 |

In some embodiments of the present disclosure, the differential scanning calorimetry curve of the crystal form B has endothermic peaks with an onset at 57.40±3.0°C and 296.86±3.0°C, respectively.

In some embodiments of the present disclosure, the DSC pattern of the crystal form B is shown in FIG. 5.

In some embodiments of the present disclosure, the thermogravimetric analysis curve of the crystal form B has a weight loss of 10.53% at 150.0±3.0°C.

In some embodiments of the present disclosure, the TGA pattern of the crystal form B is shown in FIG. 6.

The present disclosure provides a crystal form C of a compound represented by formula (II), wherein the crystal form C has an X-ray powder diffraction pattern comprising characteristic diffraction peaks at the following 2θ angles: 10.74±0.20°, 13.64±0.20°, 21.14±0.20°,

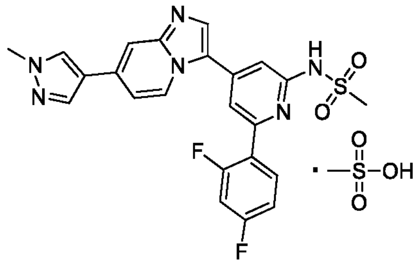

(II)

In some embodiments of the present disclosure, the X-ray powder diffraction pattern of the crystal form C has characteristic diffraction peaks at the following 2θ angles: 10.74±0.20°, 13.64±0.20°, 19.62±0.20°, 21.14±0.20°, 25.45±0.20°, 25.96±0.20°.

In some embodiments of the present disclosure, the X-ray powder diffraction pattern of the crystal form C has characteristic diffraction peaks at the following 2θ angles: 8.70±0.20°, 10.74±0.20°, 13.64±0.20°, 16.63±0.20°, 19.62±0.20°, 21.14±0.20°, 25.45±0.20°, 27.47±0.20°.

In some embodiments of the present disclosure, the X-ray powder diffraction pattern of the crystal form C has characteristic diffraction peaks at the following 2θ angles: 5.44°, 8.70°, 10.74°, 13.64°, 15.85°, 16.63°, 17.44°, 19.62°, 21.14°, 21.61°, 24.26°, 25.45°, 25.96°, 27.47°, 29.07°.

In some embodiments of the present disclosure, the XRPD pattern of the crystal form C is shown in FIG. 7.

In some embodiments of the present disclosure, the XRPD pattern analysis data of the crystal form C is shown in Table 3:

**Table 3: XRPD analysis data of the crystal form C of the compound represented by formula (II)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 5.44 | 16.26 | 16.46 | 9 | 21.14 | 4.20 | 66.36 |
| 2 | 8.70 | 10.17 | 22.44 | 10 | 21.61 | 4.11 | 19.16 |
| 3 | 10.74 | 8.24 | 100.00 | 11 | 24.26 | 3.67 | 14.43 |
| 4 | 13.64 | 6.49 | 63.75 | 12 | 25.45 | 3.50 | 65.77 |
| 5 | 15.85 | 5.59 | 12.35 | 13 | 25.96 | 3.43 | 40.52 |
| 6 | 16.63 | 5.33 | 41.88 | 14 | 27.47 | 3.25 | 28.25 |
| 7 | 17.44 | 5.09 | 12.70 | 15 | 29.07 | 3.07 | 13.48 |
| 8 | 19.62 | 4.53 | 48.17 | | | | |

In some embodiments of the present disclosure, the differential scanning calorimetry curve of the crystal form C has endothermic peaks with an onset at 37.60±3.0°C and 299.00±3.0°C, respectively.

In some embodiments of the present disclosure, the DSC pattern of the crystal form C is shown in FIG. 8.

In some embodiments of the present disclosure, the thermogravimetric analysis curve of the crystal form C has a weight loss of 7.91% at 150.0±3.0°C.

In some embodiments of the present disclosure, the TGA pattern of the crystal form C is shown in FIG. 9.

The present disclosure provides a crystal form D of a compound represented by formula (II), wherein the crystal form D has an X-ray powder diffraction pattern comprising characteristic diffraction peaks at the following 2θ angles: 10.78±0.20°, 13.64±0.20°, 16.66±0.20°,

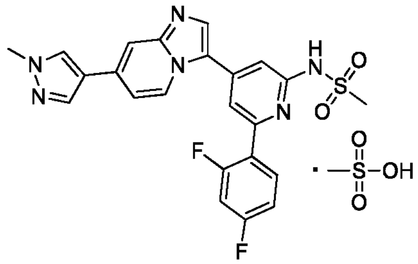

(II) .

In some embodiments of the present disclosure, the X-ray powder diffraction pattern of the crystal form D has characteristic diffraction peaks at the following 2θ angles: 10.78±0.20°, 13.64±0.20°, 16.66±0.20°, 19.63±0.20°, 21.13±0.20°, 25.40±0.20°.

In some embodiments of the present disclosure, the X-ray powder diffraction pattern of the crystal form D has characteristic diffraction peaks at the following 2θ angles: 5.51±0.20°, 8.73±0.20°, 10.78±0.20°, 13.64±0.20°, 16.66±0.20°, 19.63±0.20°, 21.13±0.20°, 25.40±0.20°.

In some embodiments of the present disclosure, the X-ray powder diffraction pattern of the crystal form D has characteristic diffraction peaks at the following 2θ angles: 5.51°, 8.10°, 8.73°, 10.78°, 12.64°, 13.64°, 14.47°, 14.92°, 15.80°, 16.66°, 17.47°, 19.03°, 19.63°, 21.13°, 21.69°, 22.02°, 22.20°, 23.84°, 24.31°, 25.40°, 25.93°, 26.28°, 26.84°, 27.41°, 27.93°, 29.10°, 30.01°, 30.78°, 32.16°, 32.78°, 33.57°, 38.41°.

In some embodiments of the present disclosure, the XRPD pattern of the crystal form D is shown in FIG. 10.

In some embodiments of the present disclosure, the XRPD pattern analysis data of the crystal form D is shown in Table 4:

**Table 4: XRPD analysis data of the crystal form D of the compound represented by formula (II)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 5.51 | 16.04 | 35.67 | 17 | 22.20 | 4.00 | 10.30 |
| 2 | 8.10 | 10.91 | 8.61 | 18 | 23.84 | 3.73 | 11.65 |
| 3 | 8.73 | 10.12 | 43.60 | 19 | 24.31 | 3.66 | 19.29 |
| 4 | 10.78 | 8.21 | 100.00 | 20 | 25.40 | 3.51 | 57.02 |
| 5 | 12.64 | 7.00 | 11.03 | 21 | 25.93 | 3.44 | 41.81 |
| 6 | 13.64 | 6.49 | 84.82 | 22 | 26.28 | 3.39 | 24.77 |
| 7 | 14.47 | 6.12 | 11.19 | 23 | 26.84 | 3.32 | 9.54 |
| 8 | 14.92 | 5.94 | 13.85 | 24 | 27.41 | 3.25 | 28.58 |
| 9 | 15.80 | 5.61 | 12.88 | 25 | 27.93 | 3.19 | 12.88 |
| 10 | 16.66 | 5.32 | 93.21 | 26 | 29.10 | 3.07 | 18.40 |
| 11 | 17.47 | 5.08 | 26.96 | 27 | 30.01 | 2.98 | 2.05 |
| 12 | 19.03 | 4.66 | 10.08 | 28 | 30.78 | 2.90 | 2.38 |
| 13 | 19.63 | 4.52 | 72.43 | 29 | 32.16 | 2.78 | 2.91 |
| 14 | 21.13 | 4.21 | 69.61 | 30 | 32.78 | 2.73 | 4.65 |
| 15 | 21.69 | 4.10 | 30.75 | 31 | 33.57 | 2.67 | 2.53 |
| 16 | 22.02 | 4.04 | 17.52 | 32 | 38.41 | 2.34 | 1.98 |

In some embodiments of the present disclosure, the differential scanning calorimetry curve of the crystal form D has endothermic peaks with an onset at 27.1±3.0°C and 298.8±3.0°C, respectively.

In some embodiments of the present disclosure, the DSC pattern of the crystal form D is shown in FIG. 11.

In some embodiments of the present disclosure, the thermogravimetric analysis curve of the crystal form D has

a weight loss of 3.15% at 150.0±3,0°C.

In some embodiments of the present disclosure, the TGA pattern of the crystal form D is shown in FIG. 12.

The present disclosure provides a compound represented by formula (III),

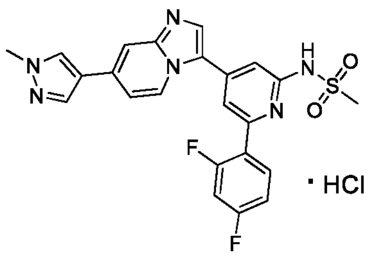

(III)

The present disclosure provides a crystal form E of a compound represented by formula (III), wherein the crystal form E has an X-ray powder diffraction pattern comprising characteristic diffraction peaks at the following 2θ angles: 9.56±0.20°, 19.10±0.20°, 27.12±0.20°,

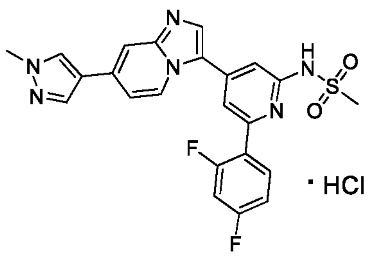

(III)

In some embodiments of the present disclosure, the X-ray powder diffraction pattern of the crystal form E has characteristic diffraction peaks at the following 2θ angles: 9.56±0.20°, 10.82±0.20°, 16.94±0.20°, 19.10±0.20°, 27.12±0.20°, 28.76±0.20°.

In some embodiments of the present disclosure, the X-ray powder diffraction pattern of the crystal form E has characteristic diffraction peaks at the following 2θ angles: 9.56±0.20°, 10.82±0.20°, 16.94±0.20°, 17.57±0.20°, 19.10±0.20°, 25.00±0.20°, 27.12±0.20°, 28.76±0.20°.

In some embodiments of the present disclosure, the X-ray powder diffraction pattern of the crystal form E has characteristic diffraction peaks at the following 2θ angles: 7.01°, 9.56°, 10.82°, 13.51°, 13.97°, 16.94°, 17.57°, 19.10°, 21.26°, 23.73°, 24.47°, 25.00°, 26.04°, 26.62°, 27.12°, 28.33°, 28.76°, 29.22°, 30.59°, 31.56°, 32.72°, 35.31°, 36.10°, 37.25°, 38.64°.

In some embodiments of the present disclosure, the XRPD pattern of the crystal form E is shown in FIG. 13.

In some embodiments of the present disclosure, the XRPD pattern analysis data of the crystal form E is shown in Table 5:

**Table 5: XRPD analysis data of the crystal form E of the compound represented by formula (III)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 7.01 | 12.62 | 1.83 | 14 | 26.62 | 3.35 | 3.05 |
| 2 | 9.56 | 9.25 | 100.00 | 15 | 27.12 | 3.29 | 9.60 |
| 3 | 10.82 | 8.18 | 6.05 | 16 | 28.33 | 3.15 | 1.36 |
| 4 | 13.51 | 6.55 | 2.25 | 17 | 28.76 | 3.10 | 4.51 |
| 5 | 13.97 | 6.34 | 2.34 | 18 | 29.22 | 3.06 | 2.46 |
| 6 | 16.94 | 5.23 | 4.30 | 19 | 30.59 | 2.92 | 1.02 |
| 7 | 17.57 | 5.05 | 3.05 | 20 | 31.56 | 2.83 | 2.54 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 8 | 19.10 | 4.65 | 38.36 | 21 | 32.72 | 2.74 | 0.69 |
| 9 | 21.26 | 4.18 | 2.38 | 22 | 35.31 | 2.54 | 0.74 |
| 10 | 23.73 | 3.75 | 2.07 | 23 | 36.10 | 2.49 | 2.15 |
| 11 | 24.47 | 3.64 | 1.10 | 24 | 37.25 | 2.41 | 0.34 |
| 12 | 25.00 | 3.56 | 3.39 | 25 | 38.64 | 2.33 | 5.18 |
| 13 | 26.04 | 3.42 | 2.65 | | | | |

In some embodiments of the present disclosure, the differential scanning calorimetry curve of the crystal form E has an endothermic peak with an onset at 303.8±3.0°C.

In some embodiments of the present disclosure, the DSC pattern of the crystal form E is shown in FIG. 14.

In some embodiments of the present disclosure, the thermogravimetric analysis curve of the crystal form E has a weight loss of 2.27% at 200.0±3.0°C.

In some embodiments of the present disclosure, the TGA pattern of the crystal form E is shown in FIG. 15.

The present disclosure provides a crystal form F of a compound represented by formula (III), wherein the crystal form F has an X-ray powder diffraction pattern comprising characteristic diffraction peaks at the following 2θ angles: 8.08±0.20°, 19.09±0.20°, 26.87±0.20°,

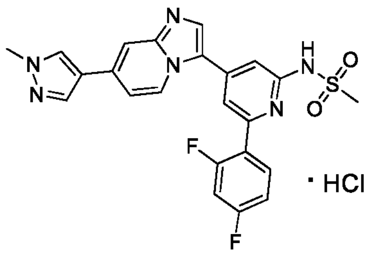

(III)

In some embodiments of the present disclosure, the X-ray powder diffraction pattern of the crystal form F has characteristic diffraction peaks at the following 2θ angles: 8.08±0.20°, 9.51±0.20°, 12.40±0.20°, 19.09±0.20°, 24.91±0.20°, 26.87±0.20°.

In some embodiments of the present disclosure, the X-ray powder diffraction pattern of the crystal form F has characteristic diffraction peaks at the following 2θ angles: 8.08±0.20°, 9.51±0.20°, 12.40±0.20°, 16.80±0.20°, 17.70±0.20°, 19.09±0.20°, 24.91±0.20°, 26.87±0.20°.

In some embodiments of the present disclosure, the X-ray powder diffraction pattern of the crystal form F has characteristic diffraction peaks at the following 2θ angles: 8.08°, 9.51°, 12.40°, 13.34°, 14.53°, 16.80°, 17.70°, 19.09°, 20.34°, 22.34°, 24.91°, 26.87°, 28.87°.

In some embodiments of the present disclosure, the XRPD pattern of the crystal form F is shown in FIG. 16.

In some embodiments of the present disclosure, the XRPD pattern analysis data of the crystal form F is shown in Table 6:

**Table 6: XRPD analysis data of the crystal form F of the compound represented by formula (III)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 8.08 | 10.94 | 75.38 | 8 | 19.09 | 4.65 | 64.79 |
| 2 | 9.51 | 9.30 | 60.10 | 9 | 20.34 | 4.37 | 16.75 |
| 3 | 12.40 | 7.14 | 36.24 | 10 | 22.34 | 3.98 | 5.71 |
| 4 | 13.34 | 6.64 | 5.31 | 11 | 24.91 | 3.57 | 55.19 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 5 | 14.53 | 6.10 | 11.63 | 12 | 26.87 | 3.32 | 100.00 |
| 6 | 16.80 | 5.28 | 23.15 | 13 | 28.87 | 3.09 | 6.87 |
| 7 | 17.70 | 5.01 | 36.08 | 14 | | | |

The present disclosure provides a crystal form G of a compound represented by formula (III), wherein the crystal form G has an X-ray powder diffraction pattern comprising characteristic diffraction peaks at the following 2θ angles: 8.79±0.20°, 17.53±0.20°, 26.33±0.20°,

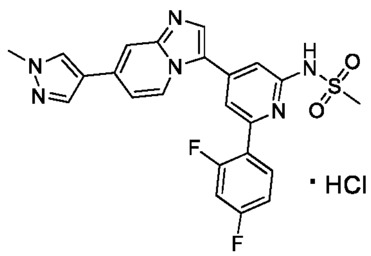

(III)

In some embodiments of the present disclosure, the X-ray powder diffraction pattern of the crystal form G has characteristic diffraction peaks at the following 2θ angles: 8.79±0.20°, 12.34±0.20°, 17.53±0.20°, 19.10±0.20°, 25.16±0.20°, 26.33±0.20°.

In some embodiments of the present disclosure, the X-ray powder diffraction pattern of the crystal form G has characteristic diffraction peaks at the following 2θ angles: 8.79±0.20°, 12.34±0.20°, 17.53±0.20°, 19.10±0.20°, 19.65±0.20°, 21.45±0.20°, 25.16±0.20°, 26.33±0.20°.

In some embodiments of the present disclosure, the X-ray powder diffraction pattern of the crystal form G has characteristic diffraction peaks at the following 2θ angles: 8.79°, 12.34°, 13.92°, 15.13°, 15.76°, 17.08°, 17.53°, 19.10°, 19.65°, 20.61°, 21.45°, 21.90°, 23.38°, 25.16°, 26.33°, 26.70°, 29.18°, 35.42°, 37.62°.

In some embodiments of the present disclosure, the XRPD pattern of the crystal form G is shown in FIG. 17.

In some embodiments of the present disclosure, the XRPD pattern analysis data of the crystal form G is shown in Table 7:

**Table 7: XRPD analysis data of the crystal form G of the compound represented by formula (III)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 8.79 | 10.06 | 100.00 | 11 | 21.45 | 4.14 | 7.04 |
| 2 | 12.34 | 7.17 | 18.32 | 12 | 21.90 | 4.06 | 4.45 |
| 3 | 13.92 | 6.36 | 3.75 | 13 | 23.38 | 3.80 | 5.80 |
| 4 | 15.13 | 5.86 | 4.52 | 14 | 25.16 | 3.54 | 12.24 |
| 5 | 15.76 | 5.62 | 2.75 | 15 | 26.33 | 3.39 | 25.11 |
| 6 | 17.08 | 5.19 | 6.13 | 16 | 26.70 | 3.34 | 12.92 |
| 7 | 17.53 | 5.06 | 66.85 | 17 | 29.18 | 3.06 | 2.29 |
| 8 | 19.10 | 4.65 | 8.51 | 18 | 35.42 | 2.53 | 0.92 |
| 9 | 19.65 | 4.52 | 7.85 | 19 | 37.62 | 2.39 | 0.83 |
| 10 | 20.61 | 4.31 | 4.03 | | | | |

The present disclosure provides a compound represented by formula (IV),

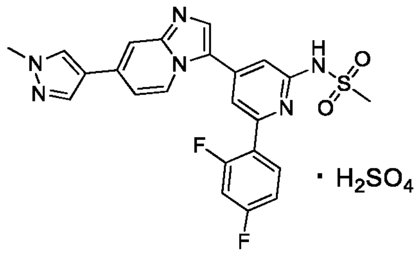

(IV)

.

The present disclosure provides a crystal form H of a compound represented by formula (IV), wherein the crystal form H has an X-ray powder diffraction pattern comprising characteristic diffraction peaks at the following 2θ angles: 6.65±0.20°, 17.80±0.20°, 18.92±0.20°,

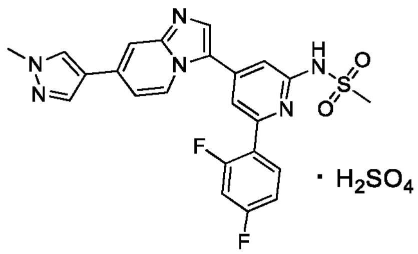

(IV)

.

In some embodiments of the present disclosure, the X-ray powder diffraction pattern of the crystal form H has characteristic diffraction peaks at the following 2θ angles: 6.65±0.20°, 13.42±0.20°, 17.80±0.20°, 18.92±0.20°, 21.99±0.20°, 24.42±0.20°.

In some embodiments of the present disclosure, the X-ray powder diffraction pattern of the crystal form H has characteristic diffraction peaks at the following 2θ angles: 6.65±0.20°, 13.42±0.20°, 17.80±0.20°, 18.92±0.20°, 20.05±0.20°, 21.99±0.20°, 24.42±0.20°, 26.30±0.20°.

In some embodiments of the present disclosure, the X-ray powder diffraction pattern of the crystal form H has characteristic diffraction peaks at the following 2θ angles: 6.65°, 13.42°, 17.80°, 18.45°, 18.92°, 20.05°, 21.99°, 24.42°, 26.30°, 27.00°.

In some embodiments of the present disclosure, the XRPD pattern of the crystal form H is shown in FIG. 18.

In some embodiments of the present disclosure, the XRPD pattern analysis data of the crystal form H is shown in Table 8:

**Table 8: XRPD analysis data of the crystal form H of the compound represented by formula (IV)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.65 | 13.29 | 100.00 | 6 | 20.05 | 4.43 | 11.22 |
| 2 | 13.42 | 6.60 | 13.68 | 7 | 21.99 | 4.04 | 18.53 |
| 3 | 17.80 | 4.98 | 56.55 | 8 | 24.42 | 3.65 | 37.47 |
| 4 | 18.45 | 4.81 | 1.72 | 9 | 26.30 | 3.39 | 12.56 |
| 5 | 18.92 | 4.69 | 41.39 | 10 | 27.00 | 3.30 | 10.52 |

In some embodiments of the present disclosure, the differential scanning calorimetry curve of the crystal form H has endothermic peaks with an onset at 275.73±3.0°C and 310.54±3.0°C, respectively.

In some embodiments of the present disclosure, the DSC pattern of the crystal form H is shown in FIG. 19.

In some embodiments of the present disclosure, the thermogravimetric analysis curve of the crystal form H has a weight loss of 8.58% at 200.0±3.0°C and a weight loss of 2.45% at 260.0±3.0°C.

In some embodiments of the present disclosure, the TGA pattern of the crystal form H is shown in FIG. 20.

The present disclosure provides a crystal form I of a compound represented by formula (IV), wherein the crystal form I has an X-ray powder diffraction pattern comprising characteristic diffraction peaks at the following 2θ angles: 5.33±0.20°, 13.74±0.20°, 20.66±0.20°,

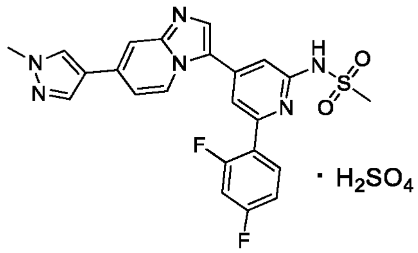

(IV)

In some embodiments of the present disclosure, the X-ray powder diffraction pattern of the crystal form I has characteristic diffraction peaks at the following 2θ angles: 5.33±0.20°, 9.02±0.20°, 13.74±0.20°, 18.16±0.20°, 20.66±0.20°, 21.91±0.20°.

In some embodiments of the present disclosure, the X-ray powder diffraction pattern of the crystal form I has characteristic diffraction peaks at the following 2θ angles: 5.33±0.20°, 9.02±0.20°, 11.77±0.20°, 13.74±0.20°, 17.51±0.20°, 18.16±0.20°, 20.66±0.20°, 21.91±0.20°.

In some embodiments of the present disclosure, the X-ray powder diffraction pattern of the crystal form I has characteristic diffraction peaks at the following 2θ angles: 5.33°, 9.02°, 10.62°, 11.77°, 13.74°, 15.99°, 17.51°, 18.16°, 19.63°, 20.66°, 21.24°, 21.91°, 23.15°, 24.94°, 26.89°.

In some embodiments of the present disclosure, the XRPD pattern of the crystal form I is shown in FIG. 21.

In some embodiments of the present disclosure, the XRPD pattern analysis data of the crystal form I is shown in Table 9:

**Table 9: XRPD analysis data of the crystal form I of the compound represented by formula (IV)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 5.33 | 16.58 | 100.00 | 9 | 19.63 | 4.52 | 7.07 |
| 2 | 9.02 | 9.80 | 11.96 | 10 | 20.66 | 4.30 | 40.11 |
| 3 | 10.62 | 8.33 | 8.32 | 11 | 21.24 | 4.18 | 2.86 |
| 4 | 11.77 | 7.52 | 10.31 | 12 | 21.91 | 4.06 | 26.19 |
| 5 | 13.74 | 6.45 | 52.17 | 13 | 23.15 | 3.84 | 9.81 |
| 6 | 15.99 | 5.54 | 5.39 | 14 | 24.94 | 3.57 | 9.04 |
| 7 | 17.51 | 5.06 | 12.67 | 15 | 26.89 | 3.32 | 7.14 |
| 8 | 18.16 | 4.89 | 37.32 | | | | |

The present disclosure provides a crystal form J of a compound represented by formula (IV), wherein the crystal form J has an X-ray powder diffraction pattern comprising characteristic diffraction peaks at the following 2θ angles: 5.24±0.20°, 18.48±0.20°, 20.79±0.20°,

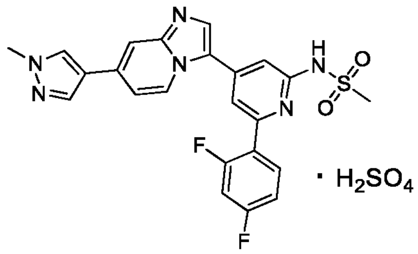

(IV)

.

In some embodiments of the present disclosure, the X-ray powder diffraction pattern of the crystal form J has characteristic diffraction peaks at the following 2θ angles: 5.24±0.20°, 18.48±0.20°, 19.77±0.20°, 20.79±0.20°, 22.67±0.20°, 23.24±0.20°.

In some embodiments of the present disclosure, the X-ray powder diffraction pattern of the crystal form J has characteristic diffraction peaks at the following 2θ angles: 5.24±0.20°, 18.48±0.20°, 19.77±0.20°, 20.79±0.20°, 22.67±0.20°, 23.24±0.20°, 24.20±0.20°, 26.28±0.20°.

In some embodiments of the present disclosure, the X-ray powder diffraction pattern of the crystal form J has characteristic diffraction peaks at the following 2θ angles: 5.24°, 12.55°, 13.78°, 14.86°, 16.30°, 17.19°, 18.48°, 19.77°, 20.79°, 22.67°, 23.24°, 24.20°, 26.28°.

In some embodiments of the present disclosure, the XRPD pattern of the crystal form J is shown in FIG. 22.

In some embodiments of the present disclosure, the XRPD pattern analysis data of the crystal form J is shown in Table 10:

**Table 10: XRPD analysis data of the crystal form J of the compound represented by formula (IV)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 5.24 | 16.85 | 78.05 | 8 | 19.77 | 4.49 | 43.47 |
| 2 | 12.55 | 7.05 | 10.60 | 9 | 20.79 | 4.27 | 68.55 |
| 3 | 13.78 | 6.43 | 10.87 | 10 | 22.67 | 3.92 | 44.62 |
| 4 | 14.86 | 5.96 | 6.42 | 11 | 23.24 | 3.83 | 34.96 |
| 5 | 16.30 | 5.44 | 10.49 | 12 | 24.20 | 3.68 | 27.66 |
| 6 | 17.19 | 5.16 | 16.43 | 13 | 26.28 | 3.39 | 18.09 |
| 7 | 18.48 | 4.80 | 100.00 | | | | |

The present disclosure provides a crystal form K of a compound represented by formula (IV), wherein the crystal form K has an X-ray powder diffraction pattern comprising characteristic diffraction peaks at the following 2θ angles: 17.97±0.20°, 20.47±0.20°, 25.16±0.20°,

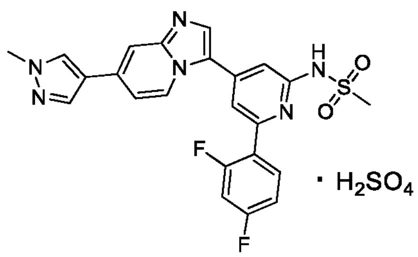

(IV)

.

In some embodiments of the present disclosure, the X-ray powder diffraction pattern of the crystal form K has characteristic diffraction peaks at the following 2θ angles: 6.79±0.20°, 17.97±0.20°, 20.47±0.20°, 23.46±0.20°, 23.87±0.20°, 25.16±0.20°.

In some embodiments of the present disclosure, the X-ray powder diffraction pattern of the crystal form K has

characteristic diffraction peaks at the following 2θ angles: 6.79±0.20°, 17.97±0.20°, 18.74±0.20°, 19.47±0.20°, 20.47±0.20°, 23.46±0.20°, 23.87±0.20°, 25.16±0.20°.

In some embodiments of the present disclosure, the X-ray powder diffraction pattern of the crystal form K has characteristic diffraction peaks at the following 2θ angles: 6.79°, 7.87°, 8.65°, 11.69°, 16.47°, 17.97°, 18.28°, 18.74°, 19.47°, 20.47°, 20.76°, 21.73°, 22.33°, 23.46°, 23.87°, 25.16°, 25.94°, 26.30°, 27.06°, 28.07°, 29.34°, 30.18°, 31.69°, 33.26°, 34.45°.

In some embodiments of the present disclosure, the XRPD pattern of the crystal form K is shown in FIG. 23.

In some embodiments of the present disclosure, the XRPD pattern analysis data of the crystal form K is shown in Table 11:

**Table 11: XRPD analysis data of the crystal form K of the compound represented by formula (IV)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.79 | 13.02 | 26.40 | 14 | 23.46 | 3.79 | 30.40 |
| 2 | 7.87 | 11.24 | 5.26 | 15 | 23.87 | 3.73 | 28.76 |
| 3 | 8.65 | 10.23 | 16.53 | 16 | 25.16 | 3.54 | 63.03 |
| 4 | 11.69 | 7.57 | 14.36 | 17 | 25.94 | 3.44 | 16.61 |
| 5 | 16.47 | 5.38 | 14.91 | 18 | 26.30 | 3.39 | 12.80 |
| 6 | 17.97 | 4.94 | 100.00 | 19 | 27.06 | 3.30 | 11.23 |
| 7 | 18.28 | 4.85 | 36.07 | 20 | 28.07 | 3.18 | 6.78 |
| 8 | 18.74 | 4.74 | 23.04 | 21 | 29.34 | 3.04 | 5.58 |
| 9 | 19.47 | 4.56 | 24.71 | 22 | 30.18 | 2.96 | 5.00 |
| 10 | 20.47 | 4.34 | 33.19 | 23 | 31.69 | 2.82 | 2.76 |
| 11 | 20.76 | 4.28 | 16.83 | 24 | 33.26 | 2.69 | 2.35 |
| 12 | 21.73 | 4.09 | 8.61 | 25 | 34.45 | 2.60 | 1.42 |
| 13 | 22.33 | 3.98 | 10.95 | | | | |

The present disclosure provides a compound represented by formula (V),

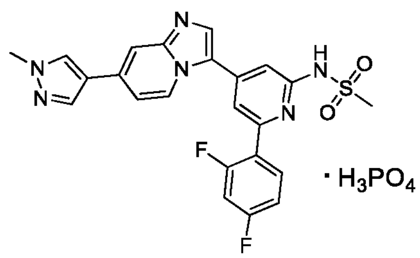

(V)

The present disclosure provides a crystal form L of a compound represented by formula (V), wherein the crystal form L has an X-ray powder diffraction pattern comprising characteristic diffraction peaks at the following 2θ angles: 5.85±0.20°, 16.75±0.20°, 20.67±0.20°,

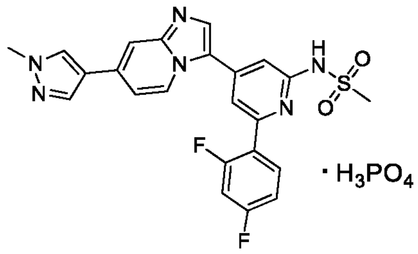

(V)

In some embodiments of the present disclosure, the X-ray powder diffraction pattern of the crystal form L has characteristic diffraction peaks at the following 2θ angles: 5.85±0.20°, 11.57±0.20°, 16.75±0.20°, 20.67±0.20°, 22.50±0.20°, 25.29±0.20°.

In some embodiments of the present disclosure, the X-ray powder diffraction pattern of the crystal form L has characteristic diffraction peaks at the following 2θ angles: 5.85±0.20°, 11.57±0.20°, 16.75±0.20°, 18.10±0.20°, 20.67±0.20°, 22.50±0.20°, 23.34±0.20°, 25.29±0.20°.

In some embodiments of the present disclosure, the X-ray powder diffraction pattern of the crystal form L has characteristic diffraction peaks at the following 2θ angles: 5.85°, 8.36°, 11.57°, 16.75°, 18.10°, 20.67°, 22.50°, 23.34°, 25.29°, 27.93°, 31.76°.

In some embodiments of the present disclosure, the XRPD pattern of the crystal form L is shown in FIG. 24.

In some embodiments of the present disclosure, the XRPD pattern analysis data of the crystal form L is shown in Table 12:

**Table 12: XRPD analysis data of the crystal form L of the compound represented by formula (V)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 5.85 | 15.10 | 18.65 | 7 | 22.50 | 3.95 | 5.65 |
| 2 | 8.36 | 10.58 | 3.30 | 8 | 23.34 | 3.81 | 4.12 |
| 3 | 11.57 | 7.65 | 8.09 | 9 | 25.29 | 3.52 | 6.97 |
| 4 | 16.75 | 5.29 | 100.00 | 10 | 27.93 | 3.19 | 3.86 |
| 5 | 18.10 | 4.90 | 5.59 | 11 | 31.76 | 2.82 | 1.85 |
| 6 | 20.67 | 4.30 | 9.95 | | | | |

The present disclosure provides a crystal form M of a compound represented by formula (V), wherein the crystal form M has an X-ray powder diffraction pattern comprising characteristic diffraction peaks at the following 2θ angles: 5.72±0.20°, 16.77±0.20°, 17.51±0.20°,

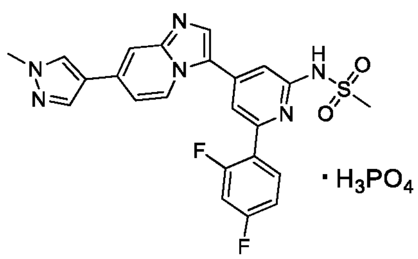

(V)

In some embodiments of the present disclosure, the X-ray powder diffraction pattern of the crystal form M has characteristic diffraction peaks at the following 2θ angles: 5.72±0.20°, 11.52±0.20°, 16.77±0.20°, 17.51±0.20°, 18.10±0.20°, 20.05±0.20°.

In some embodiments of the present disclosure, the X-ray powder diffraction pattern of the crystal form M has characteristic diffraction peaks at the following 2θ angles: 5.72±0.20°, 11.52±0.20°, 16.77±0.20°, 17.51±0.20°,

18.10±0.20°, 20.05±0.20°, 22.48±0.20°, 25.30±0.20°.

In some embodiments of the present disclosure, the X-ray powder diffraction pattern of the crystal form M has characteristic diffraction peaks at the following 2θ angles: 5.42°, 5.72°, 11.52°, 13.57°, 14.92°, 16.77°, 17.51°, 18.10°, 20.05°, 22.48°, 23.35°, 23.91°, 25.30°, 27.10°, 27.94°, 30.00°, 31.77°.

In some embodiments of the present disclosure, the XRPD pattern of the crystal form M is shown in FIG. 25.

In some embodiments of the present disclosure, the XRPD pattern analysis data of the crystal form M is shown in Table 13:

**Table 13: XRPD analysis data of the crystal form M of the compound represented by formula (V)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 5.42 | 16.30 | 14.45 | 10 | 22.48 | 3.95 | 7.09 |
| 2 | 5.72 | 15.44 | 24.25 | 11 | 23.35 | 3.81 | 4.91 |
| 3 | 11.52 | 7.68 | 18.90 | 12 | 23.91 | 3.72 | 3.03 |
| 4 | 13.57 | 6.53 | 3.02 | 13 | 25.30 | 3.52 | 7.28 |
| 5 | 14.92 | 5.94 | 1.39 | 14 | 27.10 | 3.29 | 6.59 |
| 6 | 16.77 | 5.29 | 100.00 | 15 | 27.94 | 3.19 | 4.14 |
| 7 | 17.51 | 5.06 | 19.56 | 16 | 30.00 | 2.98 | 2.23 |
| 8 | 18.10 | 4.90 | 10.22 | 17 | 31.77 | 2.82 | 1.78 |
| 9 | 20.05 | 4.43 | 7.99 | | | | |

The present disclosure provides a crystal form N of a compound represented by formula (V), wherein the crystal form N has an X-ray powder diffraction pattern comprising characteristic diffraction peaks at the following 2θ angles: 4.55±0.20°, 16.76±0.20°, 18.30±0.20°,

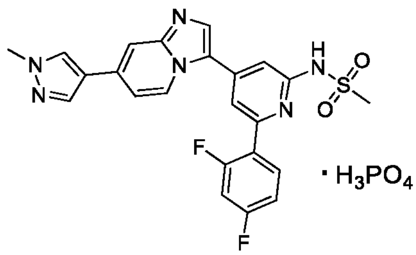

(V) .

In some embodiments of the present disclosure, the X-ray powder diffraction pattern of the crystal form N has characteristic diffraction peaks at the following 2θ angles: 4.55±0.20°, 16.14±0.20°, 16.76±0.20°, 17.20±0.20°, 18.30±0.20°, 20.22±0.20°.

In some embodiments of the present disclosure, the X-ray powder diffraction pattern of the crystal form N has characteristic diffraction peaks at the following 2θ angles: 4.55±0.20°, 16.14±0.20°, 16.76±0.20°, 17.20±0.20°, 18.30±0.20°, 20.22±0.20°, 22.63±0.20°, 24.50±0.20°.

In some embodiments of the present disclosure, the X-ray powder diffraction pattern of the crystal form N has characteristic diffraction peaks at the following 2θ angles: 4.55°, 16.14°, 16.76°, 17.20°, 18.30°, 20.22°, 22.63°, 24.50°, 26.73°, 31.76°.

In some embodiments of the present disclosure, the XRPD pattern of the crystal form N is shown in FIG. 26.

In some embodiments of the present disclosure, the XRPD pattern analysis data of the crystal form N is shown in Table 14:

**Table 14: XRPD analysis data of the crystal form N of the compound represented by formula (V)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 4.55 | 19.42 | 100.00 | 6 | 20.22 | 4.39 | 24.52 |
| 2 | 16.14 | 5.49 | 20.45 | 7 | 22.63 | 3.93 | 10.77 |
| 3 | 16.76 | 5.29 | 55.85 | 8 | 24.50 | 3.63 | 14.73 |
| 4 | 17.20 | 5.15 | 17.03 | 9 | 26.73 | 3.34 | 6.60 |
| 5 | 18.30 | 4.85 | 64.59 | 10 | 31.76 | 2.82 | 6.50 |

The present disclosure provides a compound represented by formula (VI),

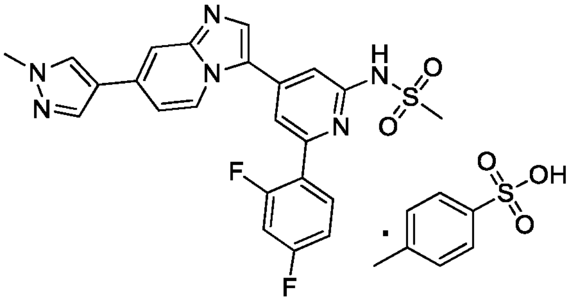

(VI)

.

The present disclosure provides a crystal form O of a compound represented by formula (VI), wherein t the crystal form O has an X-ray powder diffraction pattern comprising characteristic diffraction peaks at the following 2θ angles: 4.57±0.20°, 5.79±0.20°, 18.06±0.20°,

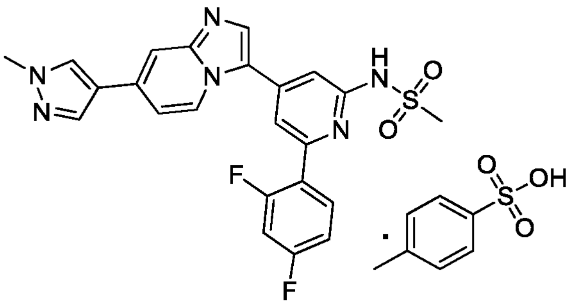

(VI)

.

In some embodiments of the present disclosure, the X-ray powder diffraction pattern of the crystal form O has characteristic diffraction peaks at the following 2θ angles: 4.57±0.20°, 5.79±0.20°, 16.38±0.20°, 18.06±0.20°, 19.32±0.20°, 20.13±0.20°.

In some embodiments of the present disclosure, the X-ray powder diffraction pattern of the crystal form O has characteristic diffraction peaks at the following 2θ angles: 4.57±0.20°, 5.79±0.20°, 9.09±0.20°, 14.52±0.20°, 16.38±0.20°, 18.06±0.20°, 19.32±0.20°, 20.13±0.20°.

In some embodiments of the present disclosure, the X-ray powder diffraction pattern of the crystal form O has characteristic diffraction peaks at the following 2θ angles: 4.57°, 5.79°, 6.45°, 9.09°, 10.09°, 12.20°, 13.04°, 14.52°, 16.38°, 18.06°, 18.33°, 19.32°, 20.13°, 22.42°, 22.74°, 23.32°, 23.90°, 27.37°, 29.29°.

In some embodiments of the present disclosure, the XRPD pattern of the crystal form O is shown in FIG. 27.

In some embodiments of the present disclosure, the XRPD pattern analysis data of the crystal form O is shown in Table 15:

**Table 15: XRPD analysis data of the crystal form O of the compound represented by formula (VI)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 4.57 | 19.34 | 100.00 | 11 | 18.33 | 4.84 | 23.82 |
| 2 | 5.79 | 15.27 | 59.42 | 12 | 19.32 | 4.59 | 11.14 |
| 3 | 6.45 | 13.70 | 0.79 | 13 | 20.13 | 4.41 | 11.14 |
| 4 | 9.09 | 9.72 | 7.01 | 14 | 22.42 | 3.97 | 4.17 |
| 5 | 10.09 | 8.76 | 4.08 | 15 | 22.74 | 3.91 | 2.73 |
| 6 | 12.20 | 7.26 | 3.07 | 16 | 23.32 | 3.81 | 5.20 |
| 7 | 13.04 | 6.79 | 1.20 | 17 | 23.90 | 3.72 | 3.68 |
| 8 | 14.52 | 6.10 | 6.08 | 18 | 27.37 | 3.26 | 1.72 |
| 9 | 16.38 | 5.41 | 8.27 | 19 | 29.29 | 3.05 | 2.62 |
| 10 | 18.06 | 4.91 | 37.26 | | | | |

The present disclosure provides a crystal form P of a compound represented by formula (VI), wherein the crystal form P has an X-ray powder diffraction pattern comprising characteristic diffraction peaks at the following 2θ angles: 9.96±0.20°, 17.02±0.20°, 21.78±0.20°,

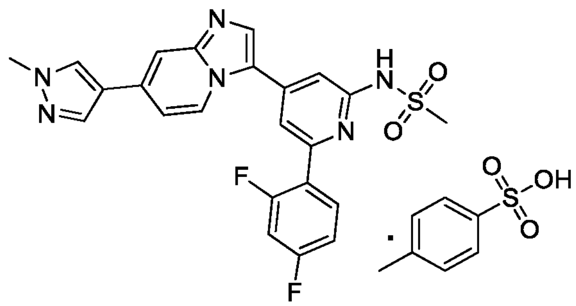

(VI)

In some embodiments of the present disclosure, the X-ray powder diffraction pattern of the crystal form P has characteristic diffraction peaks at the following 2θ angles: 9.96±0.20°, 17.02±0.20°, 21.31±0.20°, 21.78±0.20°, 24.71±0.20°, 25.52±0.20°.

In some embodiments of the present disclosure, the X-ray powder diffraction pattern of the crystal form P has characteristic diffraction peaks at the following 2θ angles: 9.96±0.20°, 16.23±0.20°, 17.02±0.20°, 17.81±0.20°, 21.31±0.20°, 21.78±0.20°, 24.71±0.20°, 25.52±0.20°.

In some embodiments of the present disclosure, the X-ray powder diffraction pattern of the crystal form P has characteristic diffraction peaks at the following 2θ angles: 6.87°, 8.26°, 9.96°, 13.64°, 15.18°, 16.23°, 17.02°, 17.81°, 18.62°, 21.31°, 21.78°, 24.71°, 25.52°, 29.14°, 31.47°.

In some embodiments of the present disclosure, the XRPD pattern of the crystal form P is shown in FIG. 28.

In some embodiments of the present disclosure, the XRPD pattern analysis data of the crystal form P is shown in Table 16:

**Table 16: XRPD analysis data of the crystal form P of the compound represented by formula (VI)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.87 | 12.86 | 9.82 | 9 | 18.62 | 4.76 | 11.87 |
| 2 | 8.26 | 10.70 | 7.09 | 10 | 21.31 | 4.17 | 32.01 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 3 | 9.96 | 8.88 | 100.00 | 11 | 21.78 | 4.08 | 48.65 |
| 4 | 13.64 | 6.49 | 4.96 | 12 | 24.71 | 3.60 | 29.81 |
| 5 | 15.18 | 5.84 | 6.73 | 13 | 25.52 | 3.49 | 17.69 |
| 6 | 16.23 | 5.46 | 13.58 | 14 | 29.14 | 3.06 | 5.80 |
| 7 | 17.02 | 5.21 | 44.17 | 15 | 31.47 | 2.84 | 4.56 |
| 8 | 17.81 | 4.98 | 14.86 | | | | |

The present disclosure provides a compound represented by formula (VII),

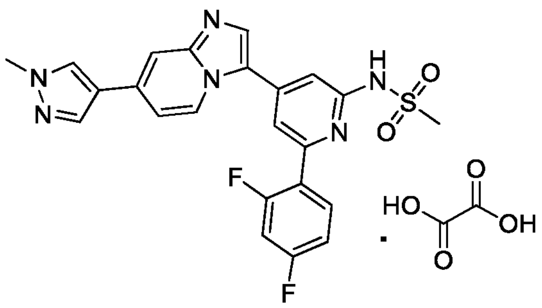

(VII)

The present disclosure provides a crystal form Q of a compound represented by formula (VII), wherein the crystal form Q has an X-ray powder diffraction pattern comprising characteristic diffraction peaks at the following 2θ angles: 7.90±0.20°, 16.76±0.20°, 25.94±0.20°,

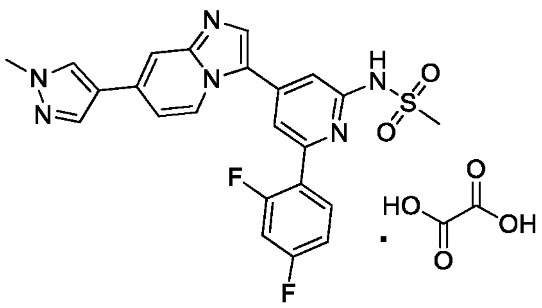

(VII)

In some embodiments of the present disclosure, the X-ray powder diffraction pattern of the crystal form Q has characteristic diffraction peaks at the following 2θ angles: 7.90±0.20°, 16.76±0.20°, 17.19±0.20°, 20.09±0.20°, 23.82±0.20°, 25.94±0.20°.

In some embodiments of the present disclosure, the X-ray powder diffraction pattern of the crystal form Q has characteristic diffraction peaks at the following 2θ angles: 7.90±0.20°, 11.53±0.20°, 16.76±0.20°, 17.19±0.20°, 20.09±0.20°, 20.94±0.20°, 23.82±0.20°, 25.94±0.20°.

In some embodiments of the present disclosure, the X-ray powder diffraction pattern of the crystal form Q has characteristic diffraction peaks at the following 2θ angles: 7.90°, 8.53°, 9.90°, 11.53°, 12.98°, 15.12°, 16.76°, 17.19°, 19.68°, 20.09°, 20.94°, 22.50°, 22.86°, 23.82°, 25.32°, 25.94°, 27.16°, 27.83°, 29.17°, 30.11°, 31.83°, 33.48°.

In some embodiments of the present disclosure, the XRPD pattern of the crystal form Q is shown in FIG. 29.

In some embodiments of the present disclosure, the XRPD pattern analysis data of the crystal form Q is shown in Table 17:

**Table 17: XRPD analysis data of the crystal form Q of the compound represented by formula (VI)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 7.90 | 11.19 | 32.88 | 12 | 22.50 | 3.95 | 6.82 |
| 2 | 8.53 | 10.37 | 6.34 | 13 | 22.86 | 3.89 | 10.90 |
| 3 | 9.90 | 8.94 | 1.89 | 14 | 23.82 | 3.74 | 12.13 |
| 4 | 11.53 | 7.67 | 11.49 | 15 | 25.32 | 3.52 | 9.39 |
| 5 | 12.98 | 6.82 | 5.35 | 16 | 25.94 | 3.44 | 16.58 |
| 6 | 15.12 | 5.86 | 3.54 | 17 | 27.16 | 3.28 | 7.40 |
| 7 | 16.76 | 5.29 | 100.00 | 18 | 27.83 | 3.21 | 2.80 |
| 8 | 17.19 | 5.16 | 12.12 | 19 | 29.17 | 3.06 | 4.88 |
| 9 | 19.68 | 4.51 | 7.47 | 20 | 30.11 | 2.97 | 2.98 |
| 10 | 20.09 | 4.42 | 12.84 | 21 | 31.83 | 2.81 | 2.11 |
| 11 | 20.94 | 4.24 | 10.46 | 22 | 33.48 | 2.68 | 1.66 |

The present disclosure provides a compound represented by formula (VIII),

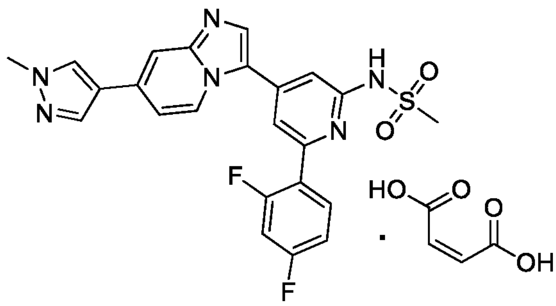

(VIII)

.

The present disclosure provides a crystal form R of a compound represented by formula (VIII), wherein the crystal form R has an X-ray powder diffraction pattern comprising characteristic diffraction peaks at the following 2θ angles: 3.18±0.20°, 6.43±0.20°, 16.67±0.20°,

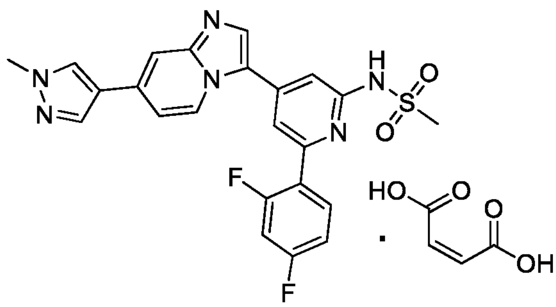

(VIII)

.

In some embodiments of the present disclosure, the X-ray powder diffraction pattern of the crystal form R has characteristic diffraction peaks at the following 2θ angles: 3.18±0.20°, 6.43±0.20°, 16.67±0.20°, 18.20±0.20°, 18.63±0.20°, 19.53±0.20°.

In some embodiments of the present disclosure, the X-ray powder diffraction pattern of the crystal form R has

characteristic diffraction peaks at the following 2θ angles: 3.18±0.20°, 6.43±0.20°, 16.67±0.20°, 18.20±0.20°, 18.63±0.20°, 19.53±0.20°, 20.02±0.20°, 27.78±0.20°.

In some embodiments of the present disclosure, the X-ray powder diffraction pattern of the crystal form R has characteristic diffraction peaks at the following 2θ angles: 3.18°, 6.43°, 11.35°, 13.24°, 16.67°, 18.20°, 18.63°, 19.53°, 20.02°, 21.59°, 23.44°, 27.78°.

In some embodiments of the present disclosure, the XRPD pattern of the crystal form R is shown in FIG. 30.

In some embodiments of the present disclosure, the XRPD pattern analysis data of the crystal form R is shown in Table 18:

**Table 18: XRPD analysis data of the crystal form R of the compound represented by formula (VIII)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 3.18 | 27.82 | 36.09 | 7 | 18.63 | 4.76 | 25.82 |
| 2 | 6.43 | 13.74 | 69.73 | 8 | 19.53 | 4.55 | 19.27 |
| 3 | 11.35 | 7.79 | 5.26 | 9 | 20.02 | 4.44 | 8.82 |
| 4 | 13.24 | 6.69 | 5.35 | 10 | 21.59 | 4.12 | 6.30 |
| 5 | 16.67 | 5.32 | 100.00 | 11 | 23.44 | 3.80 | 5.65 |
| 6 | 18.20 | 4.87 | 19.44 | 12 | 27.78 | 3.21 | 7.66 |

The present disclosure provides a compound represented by formula (X),

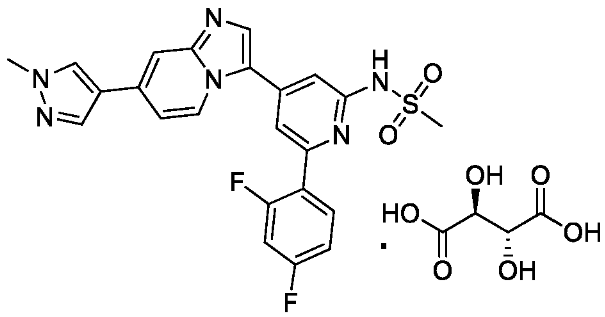

(X)

.

The present disclosure provides a crystal form S of a compound represented by formula (X), wherein the crystal form S has an X-ray powder diffraction pattern comprising characteristic diffraction peaks at the following 2θ angles: 4.74±0.20°, 17.04±0.20°, 24.77±0.20°,

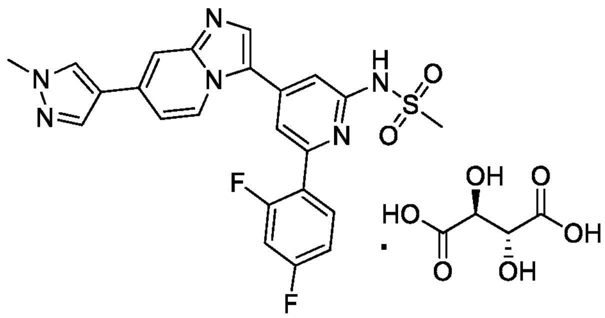

(X)

.

In some embodiments of the present disclosure, the X-ray powder diffraction pattern of the crystal form S has

characteristic diffraction peaks at the following 2θ angles: 4.74±0.20°, 11.97±0.20°, 17.04±0.20°, 20.65±0.20°, 24.77±0.20°, 31.75±0.20°.

In some embodiments of the present disclosure, the XRPD pattern of the crystal form S is shown in FIG. 31.

In some embodiments of the present disclosure, the XRPD pattern analysis data of the crystal form S is shown in Table 19:

**Table 19: XRPD analysis data of the crystal form S of the compound represented by formula (X)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 4.74 | 18.66 | 39.93 | 4 | 20.65 | 4.30 | 16.34 |
| 2 | 11.97 | 7.39 | 22.24 | 5 | 24.77 | 3.59 | 38.99 |
| 3 | 17.04 | 5.20 | 100.00 | 6 | 31.75 | 2.82 | 8.12 |

The present disclosure provides a crystal form T of a compound represented by formula (I), wherein the crystal form T has an X-ray powder diffraction pattern comprising characteristic diffraction peaks at the following 2θ angles: 8.37±0.20°, 11.54±0.20°, 16.76±0.20°,

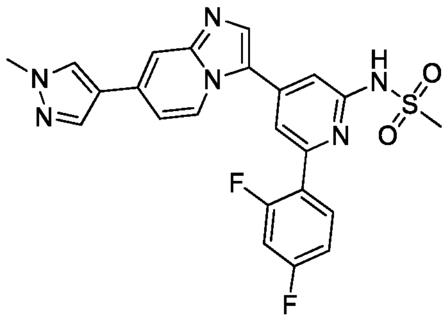

(I)

.

In some embodiments of the present disclosure, the X-ray powder diffraction pattern of the crystal form T has characteristic diffraction peaks at the following 2θ angles: 8.37±0.20°, 11.54±0.20°, 16.76±0.20°, 22.49±0.20°, 23.36±0.20°, 25.26±0.20°.

In some embodiments of the present disclosure, the X-ray powder diffraction pattern of the crystal form T has characteristic diffraction peaks at the following 2θ angles: 8.37±0.20°, 11.54±0.20°, 16.76±0.20°, 19.53±0.20°, 22.49±0.20°, 23.36±0.20°, 25.26±0.20°, 27.12±0.20°.

In some embodiments of the present disclosure, the X-ray powder diffraction pattern of the crystal form T has characteristic diffraction peaks at the following 2θ angles: 8.37°, 9.98°, 11.54°, 13.44°, 15.08°, 16.76°, 18.70°, 19.53°, 20.03°, 21.14°, 22.49°, 23.36°, 25.26°, 27.12°, 27.92°, 31.77°.

In some embodiments of the present disclosure, the XRPD pattern of the crystal form T is shown in FIG. 32.

In some embodiments of the present disclosure, the XRPD pattern analysis data of the crystal form T is shown in Table 20:

**Table 20: XRPD analysis data of the crystal form T of the compound represented by formula (I)**

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 8.37 | 10.56 | 6.37 | 9 | 20.03 | 4.43 | 1.81 |
| 2 | 9.98 | 8.87 | 1.34 | 10 | 21.14 | 4.20 | 0.55 |
| 3 | 11.54 | 7.67 | 14.01 | 11 | 22.49 | 3.95 | 4.35 |
| 4 | 13.44 | 6.59 | 0.92 | 12 | 23.36 | 3.81 | 3.36 |
| 5 | 15.08 | 5.87 | 0.70 | 13 | 25.26 | 3.53 | 2.91 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 6 | 16.76 | 5.29 | 100.00 | 14 | 27.12 | 3.29 | 2.26 |
| 7 | 18.70 | 4.74 | 1.16 | 15 | 27.92 | 3.20 | 2.19 |
| 8 | 19.53 | 4.55 | 2.41 | 16 | 31.77 | 2.82 | 1.91 |

In some embodiments of the present disclosure, the differential scanning calorimetry curve of the crystal form T has an endothermic peak with a peak value at 282.6±3.0°C.

In some embodiments of the present disclosure, the DSC pattern of the crystal form T is shown in FIG. 33.

In some embodiments of the present disclosure, the thermogravimetric analysis curve of the crystal form T has a weight loss of 1.56% at 250.0±3.0°C.

In some embodiments of the present disclosure, the TGA pattern of the crystal form T is shown in FIG. 34.

The present disclosure also provides a use of the compounds or the crystal form A, crystal form B, crystal form C, crystal form D, crystal form E, crystal form F, crystal form G, crystal form H, crystal form I, crystal form J, crystal form K, crystal form L, crystal form M, crystal form N, crystal form O, crystal form P, crystal form Q, crystal form R, crystal form S and crystal form T in the manufacture of a medicament for the treatment of diseases related to FGFR/VEGFR dual kinase inhibitors.

In some embodiments of the present disclosure, in the use, the medicament related to FGFR/VEGFR dual kinase inhibitors is a medicament for the treatment of solid tumors.

**Definition and description**

Unless otherwise specified, the following terms and phrases used herein have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

The intermediate compounds of the present disclosure can be prepared by a variety of synthetic methods known to those skilled in the art, including the preferred embodiments listed below, the embodiments formed by their combination with other chemical synthesis methods, and equivalent alternatives known to those skilled in the art, preferred embodiments include but are not limited to the embodiments of the present disclosure.

The chemical reactions of the specific embodiments of the present disclosure are completed in a suitable solvent, and the solvent must be suitable for the chemical changes of the present disclosure and the reagents and materials required for them. In order to obtain the compounds of the present disclosure, it is sometimes necessary for those skilled in the art to modify or select synthetic steps or reaction processes on the basis of existing embodiments.

The structure of the compounds of the present disclosure can be confirmed by conventional methods known to those skilled in the art, and if the present disclosure involves an absolute configuration of the compound, then the absolute configuration can be confirmed by conventional technical means in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

Unless otherwise specified, the DSC pattern used herein has a downward peak as an endothermic peak.

The following embodiments further illustrate the present disclosure, but the present disclosure is not limited thereto.

All solvents used in the present disclosure are commercially available and can be used without further purification.

The solvent used in the present disclosure is commercially available. The following abbreviations are used in the present disclosure: DCM refers to dichloromethane; DMF refers to *N*,*N*-dimethylformamide; DMSO refers to dimethyl sulfoxide; EtOH refers to ethanol; MeOH refers to methanol; TFA refers to trifluoroacetic acid; ATP refers to adenosine triphosphate; HEPES refers to 2-[4-(2-hydroxyethyl)-1-piperazinyl]ethanesulfonic acid; MgCh refers to magnesium chloride; $Pd(PPh_3)_2Cl_2$ refers to bis(triphenylphosphine)palladium(II) chloride.

**Technical effect**

The crystal forms of the compounds of the present disclosure have good stability and druggability; the crystal forms of the compounds of the present disclosure have excellent DNA-PK kinase inhibitory activity.

**X-ray powder diffractometer (XRPD) of the present disclosure**

Instrument model: X'Pert[3] X-ray diffractometer from PANalytical
Test method: Using approximately 10 mg of sample for XRPD detection.
Detailed XRPD parameters are as follows:
X-ray source: Cu, kα ($K\alpha 1$ = 1.540598 Å, $K\alpha 2$ = 1.544426 Å, $K\alpha 2/K\alpha 1$ intensity ratio: 0.5)
X-ray tube voltage: 45 kV, X-ray tube current: 40 mA
Divergence slit: fixed 1/8 deg
First seller slit: 0.04 rad, Second soller slit: 0.04 rad
Receiving slit: none, Anti-scatter slit: 7.5 mm
Measurement time: 5 min
Scanning range: 3 to 40 deg
Step width angle: 0.0263 deg
Step length: 46.665 seconds
Sample disk speed: 15 rpm

**Differential Scanning Calorimeter (DSC) of the present disclosure**

Instrument model: TA Discovery DSC 2500 Differential Scanning Calorimeter
Test method: Taking a sample (about 1 to 5 mg) and placing in a DSC aluminum disk for testing, and theating the sample from 25°C (room temperature) to a temperature before decomposition of the sample at a heating rate of 10°C/min under 50 mL/min $N_2$ conditions.

**Thermal Gravimetric Analyzer (TGA) of the present disclosure**

Instrument model: TA Discovery TGA 5500 Thermal Gravimetric Analyzer
Test method: Taking a sample (about 1 to 5 mg) and placing in a TGA aluminum disk for testing, and heating the sample from room temperature to 350°C at a heating rate of 10°C/min under 10 mL/min $N_2$ conditions.

**Dynamic Vapor Sorption (DVS) method of the present disclosure**

Instrument model: SMS Intrinsic Dynamic Vapor Sorption Analyzer
Test conditions: Taking a sample (10 to 30 mg) and placing in a DVS sample dish for testing.
Detailed DVS parameters are as follows:
Temperature: 25°C

$$\text{Equilibration: } dm/dt = 0.002\%/\text{min (minimum: 10 min, maximum: 180 min)}$$

RH range: 0% RH to 95% RH to 0% RH
RH gradient: 10% (90% RH to 0% RH to 90% RH)
5% (95% RH to 90% RH and 90% RH to 95% RH)

The classification of hygroscopicity evaluation is as follows:

| Hygroscopicity classification | ΔW% |
|---|---|
| Deliquescence | Absorption of sufficient water to form a liquid |
| Extremely hygroscopic | ΔW% ≥ 15% |
| Hygroscopic | 15% > ΔW% ≥ 2% |
| Slightly hygroscopic | 2% > ΔW% ≥ 0.2% |
| No or almost no hygroscopicity | ΔW% < 0.2% |
| Note: ΔW% represents the hygroscopic weight gain of the test sample at 25±1°C and 80±2% RH. | |

BRIEF DESCRIPTION OF THE DRAWINGS

FIG. 1 shows an XRPD pattern with Cu-Kα radiation of the crystal form A of the compound represented by formula (II);
FIG. 2 shows a DSC pattern of the crystal form A of the compound represented by formula (II);
FIG. 3 shows a TGA pattern of the crystal form A of the compound represented by formula (II);
FIG. 4 shows an XRPD pattern with Cu-Kα radiation of the crystal form B of the compound represented by formula (II);
FIG. 5 shows a DSC pattern of the crystal form B of the compound represented by formula (II);
FIG. 6 shows a TGA pattern of the crystal form B of the compound represented by formula (II);
FIG. 7 shows an XRPD pattern with Cu-Kα radiation of the crystal form C of the compound represented by formula (II);
FIG. 8 shows a DSC pattern of the crystal form C of the compound represented by formula (II);
FIG. 9 shows a TGA pattern of the crystal form C of the compound represented by formula (II);
FIG. 10 shows an XRPD pattern with Cu-Kα radiation of the crystal form D of the compound represented by formula (II);
FIG. 11 shows a DSC pattern of the crystal form D of the compound represented by formula (II);
FIG. 12 shows a TGA pattern of the crystal form D of the compound represented by formula (II);
FIG. 13 shows an XRPD pattern with Cu-Kα radiation of the crystal form E of the compound represented by formula (III);
FIG. 14 shows a DSC pattern of the crystal form E of the compound represented by formula (III);
FIG. 15 shows a TGA pattern of the crystal form E of the compound represented by formula (III);
FIG. 16 shows an XRPD pattern with Cu-Kα radiation of the crystal form F of the compound represented by formula (III);
FIG. 17 shows an XRPD pattern with Cu-Kα radiation of the crystal form G of the compound represented by formula (III);
FIG. 18 shows an XRPD pattern with Cu-Kα radiation of the crystal form H of the compound represented by formula (IV);
FIG. 19 shows a DSC pattern of the crystal form H of the compound represented by formula (IV);
FIG. 20 shows a TGA pattern of the crystal form H of the compound represented by formula (IV);
FIG. 21 shows an XRPD pattern with Cu-Kα radiation of the crystal form I of the compound represented by formula (IV);
FIG. 22 shows an XRPD pattern with Cu-Kα radiation of the crystal form J of the compound represented by formula (IV);
FIG. 23 shows an XRPD pattern with Cu-Kα radiation of the crystal form K of the compound represented by formula (IV);
FIG. 24 shows an XRPD pattern with Cu-Kα radiation of the crystal form L of the compound represented by formula (V);
FIG. 25 shows an XRPD pattern with Cu-Kα radiation of the crystal form M of the compound represented by formula (V);
FIG. 26 shows an XRPD pattern with Cu-Kα radiation of the crystal form N of the compound represented by formula (V);
FIG. 27 shows an XRPD pattern with Cu-Kα radiation of the crystal form O of the compound represented by formula (VI);
FIG. 28 shows an XRPD pattern with Cu-Kα radiation of the crystal form P of the compound represented by formula (VI);
FIG. 29 shows an XRPD pattern with Cu-Kα radiation of the crystal form Q of the compound represented by formula (VII);

FIG. 30 shows an XRPD pattern with Cu-Kα radiation of the crystal form R of the compound represented by formula (VIII);
FIG. 31 shows an XRPD pattern with Cu-Kα radiation of the crystal form S of the compound represented by formula (X);
FIG. 32 shows an XRPD pattern with Cu-Kα radiation of the crystal form T of the compound represented by formula (I);
FIG. 33 shows a DSC pattern of the crystal form T of the compound represented by formula (I);
FIG. 34 shows a TGA pattern of the crystal form T of the compound represented by formula (I);
FIG. 35 shows a DVS pattern of the crystal form A of the compound represented by formula (II).

## DETAILED DESCRIPTION OF THE PREFERRED EMBODIMENT

In order to better understand the content of the present disclosure, it will be further described below in conjunction with specific embodiments, but the specific embodiments is not a limitation to the content of the present disclosure.

### Embodiment 1: Preparation of the crystal form A of the compound represented by formula (II)

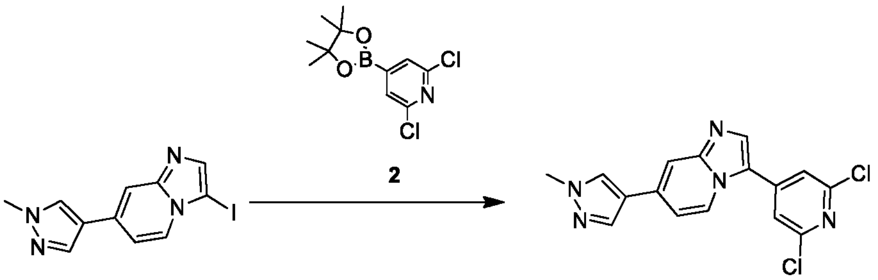
2

1 3

To a 50 L reaction kettle was added 15 L of dioxane and 5 L of water at 10 to 30°C. The reaction kettle was added with compound **1** (1500 g) and compound **2** (1335 g) at one time with stirring. The reaction kettle was added with potassium carbonate (1930 g) at one time with stirring. The reaction kettle was replaced with nitrogen for 10 min, and then added with Pd(dppf)$Cl_2$ (100 g) at one time. The reaction mixture was heated to an internal temperature of 88 to 90°C, and stirred continuously for 16 h. 10 L of the reaction mixture was added to the 50 L reaction kettle, then 30 L of water was added with stirring, and stirred at room temperature for 15 min. The remaining 10 L of the reaction mixture was treated as above. The reaction mixture was filtered under reduced pressure to obtain a filter cake. The filter cake was dried in an oven at 50°C for 48 h. Metal scavenger (1000 g), activated carbon (1000 g) and magnesium sulfate (1000 g) were added to the 50 L reaction kettle, the reaction mixture was stirred continuously at 60°C for 18 h, cooled to room temperature, and filtered through diatomite to collect a filtrate. The filtrate was concentrated to obtain a crude product. The reaction mixture was filtered under reduced pressure while hot, and the filter cake was washed with anhydrous dioxane (4 L × 2) to obtain a filtrate. The filtrate was spin-dried under reduced pressure at 40 to 50°C to obtain a residue. The concentrated crude product was transferred to the 50 L reaction kettle, the reaction mixture was added with 15 L of *n*-heptane and 1.5 L of dichloromethane respectively, and stirred at 25 to 35°C for 16 h. The suspension in the reaction kettle was aspirated into a desktop filter for filtration, and the filter cake was washed with *n*-heptane (2 L × 2) to collect a solid, which was spin-dried under reduced pressure at 40 to 50°C to obtain compound **3.**

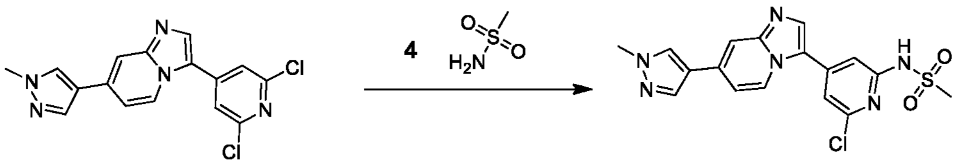
4

3 5

To a 50 L reaction flask was added 15 L of *N*,*N*-dimethylformamide at 10 to 30°C. The reaction flask was added with compound **3** (1.5 kg) and compound **4** (619.5 g) at one time with stirring, added with potassium carbonate (1.81

kg) and Xphos (415.5 g) at one time with stirring, replaced with nitrogen for 10 min, and then added with $Pd_2(dba)_3$ (399 g) at one time. The reaction mixture was heated to an internal temperature of 90 to 95°C (external temperature: 100°C), and stirred continuously for 8 h; compound 3 was 1% or less according to HPLC traces; 1 kg of diatomite was added to a desktop filter funnel, the reaction mixture was filtered under reduced pressure with the desktop filter funnel, and then washed with DMF (1 L × 2) to collect a filtrate. The filtrate was concentrated under reduced pressure to about 1/3 of the reaction volume via an oil pump. The 50 L reaction kettle was added with the above concentrated solution, then added with 3V of water, and added with 4 M sodium hydroxide aqueous solution to adjust the pH to 11 to 12. The reaction mixture was filtered under reduced pressure, and the filtrate was extracted with ethyl acetate (15 L × 2) to remove some impurities. The aqueous phase was then added with 3 M hydrochloric acid aqueous solution to adjust the pH to 5 to 6, and a large amount of yellow solid particles were precipitated. The reaction mixture was then filtered through the desktop filter funnel to collect a solid, and the filter cake was dried in a 50°C vacuum oven to obtain compound **5.**

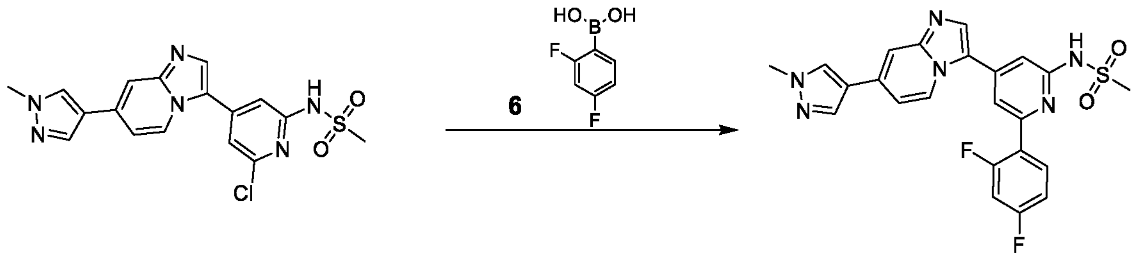


**5** **compound represented by formula (I)**

To a 50 L reaction kettle was added 11.5 L of dimethoxyethane and 3.8 L of water at 20 to 30°C. The reaction flask was added with compound **5** (1150 g) and compound **6** (676.15 g) at one time with stirring, added with potassium carbonate (1183.60 g) at one time with stirring, replaced with nitrogen for 10 min, and then added with $Pd(dppf)Cl_2$ (208.87 g) at one time. The reaction mixture was heated to an internal temperature of 77 to 80°C, and stirred continuously for 16 h; compound 5 was 1% or less according to HPLC traces; the reaction mixture was filtered under reduced pressure with the desktop filter funnel, and then washed with 1 L of dimethoxyethane to collect a filtrate. The filtrate was concentrated under reduced pressure to about 1/3 of the reaction volume via the oil pump. The 50 L reaction kettle was added with the above concentrated solution, and then added with 3V of water. The reaction mixture was added with 4 M sodium hydroxide aqueous solution to adjust the pH to 13, then added with 3 M hydrochloric acid aqueous solution to adjust the pH to 6, and a large amount of brown-yellow solid particles were precipitated. The reaction mixture was then filtered through the desktop filter funnel to collect a filter cake, and the filter cake was dried in the 50°C oven to obtain a crude product.

The 50 L reaction kettle was added with 10.0 L of DCM at 20 to 30°C, added with the crude product (1000 g), DPPE (37.94 g) and propanediamine (81.11 mL) at one time with stirring, heated to an internal temperature of 40°C, stirred continuously for 16 h, filtered under reduced pressure, and then washed with DCM (500 mL) to collect a filter cake; the above operation was repeated thrice. 3.0 L of THF was added to the 50 L reaction flask at 10 to 30°C, and the filter cake was added to the reaction kettle with stirring. The reaction mixture was heated to an internal temperature of 60°C, stirred continuously for 16 h, filtered under reduced pressure, and then washed with THF (500 mL) to collect a filter cake; the filter cake was dried under vacuum at 40 to 50°C; the 50 L reaction kettle was added with 5.0 L of DMF and 5.0 L of dioxane at 10 to 30°C, added with the crude product with stirring, heated to an internal temperature of 50°C, stirred continuously for 16 h, filtered under reduced pressure, and then washed with dioxane (500 mL) to collect a filter cake; the filter cake was dried under vacuum at 40 to 50°C; 10.0 L of THF was added to the 50 L reaction kettle at 10 to 30°C. The reaction flask was added with the filter cake with stirring, heated to an internal temperature of 60°C, stirred continuously for 16 h, filtered under reduced pressure, and then washed with THF (500 mL) to collect a filter cake. The filter cake was dried under vacuum at 40 to 50°C to obtain a crude product; the 50 L reaction kettle was added with 20.0 L of water at 10 to 30°C, added with the filter cake with stirring , then the pH was adjusted to 5 to 6, the reaction kettle was heated to 40°C, stirred continuously for 16 h, filtered under reduced pressure, and then washed with water (2 L) to collect a filter cake; the filter cake was dried under vacuum at 40 to 50°C to obtain **the compound represented by formula (I).** The compound represented by formula (I) was characterized by XRPD as a crystal form T. The XRPD pattern is shown in FIG. 32, the DSC pattern is shown in FIG. 33, and the TGA pattern is shown in FIG. 34.

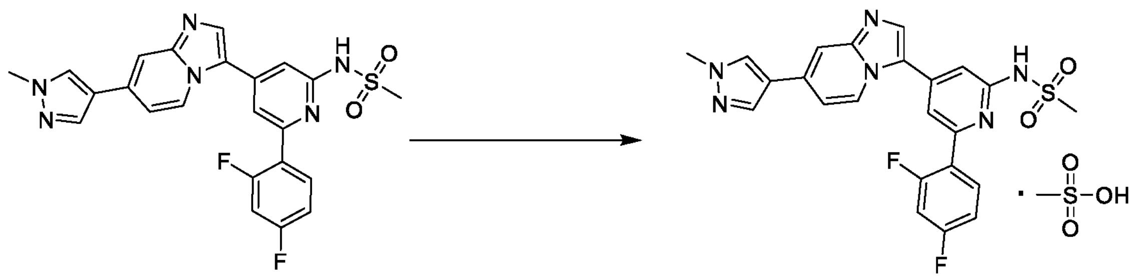

compound represented by formula (I)

crystal form A of the compound represented by formula (II)

To DMSO (7.4 L) was added the compound represented by formula (I) (740 g). The reaction mixture was added with methanesulfonic acid (155.41 g, 1.62 mol, 115.12 mL, 1.05 eq) at 20 to 30°C for reaction, stirred at 20 to 30°C for 4 h, then added with 37 L of ethyl acetate, and stirred continuously for 16 h. A large amount of solid was precipitated, filtered, the filter cake was washed with ethyl acetate (2 L × 2), and spin-dried under reduced pressure at 40 to 50°C to obtain the crystal form A of the compound represented by formula (II). The XRPD pattern of the crystal form A is shown in FIG. 1, the DSC pattern of the crystal form A is shown in FIG. 2, and the TGA pattern of the crystal form A is shown in FIG. 3.

$^1H$ NMR (400 MHz, DMSO-$d_6$) δ 8.83 (d, *J* = 4.0 Hz, 1H), 8.60 (s, 1H), 8.46 (s, 1H), 8.27 (s, 1H), 8.10 - 8.04 (m, 2H), 7.76 (s, 1H), 7.72 (d, *J* = 4.0 Hz, 1H), 7.50 - 7.32 (m, 2H), 7.24 (s, 1H), 3.93 (s, 3H), 3.47 (s, 3H), 2.30(s, 3H).

**Embodiment 2:**

**Preparation of the crystal form B of the compound represented by formula (II)**

20 mg of the compound represented by formula (I) and methanesulfonic acid (1 eq) were weighed and added to an HPLC vial respectively, mixed and added with 0.5 mL of acetone. The reaction mixture was stirred at room temperature for 4 days, centrifuged, and the solid was transferred to a vacuum dryer at 50°C for 0.5 h to obtain the crystal form B of the compound represented by formula (II).

**Preparation of the crystal form C of the compound represented by formula (II)**

20 mg of the compound represented by formula (I) and methanesulfonic acid (1 eq) were weighed and added to an HPLC vial respectively, mixed and added with 0.5 mL of EtOH/$H_2O$ (19:1, v/v). The reaction mixture was stirred at room temperature for 4 days, centrifuged, and the solid was transferred to a vacuum dryer at 50°C for 0.5 h to obtain the crystal form C of the compound represented by formula (II).

**Preparation of the crystal form D of the compound represented by formula (II)**

To DMSO (750 mL) was added the compound represented by formula (I) (75 g). The reaction mixture was added with methanesulfonic acid (15 g, 1 eq) at 20 to 30°C to dissolve, stirred for 4 h, then added with 1.5 L of ethyl acetate, and stirred for 20 h. A solid was precipitated, filtered, the filter cake was washed with ethyl acetate (50 mL × 2), and concentrated to dryness to obtain a crude product (about 45 g). The crude product was added to ethanol (450 mL), the reaction mixture was stirred at 20 to 30°C for 24 h, and filtered. The filter cake was washed with ethanol (10 mL × 2), and spin-dried under reduced pressure at 40 to 50°C to obtain the crystal form D of the compound represented by formula (II).

$^1H$ NMR (400 MHz, DMSO-$d_6$) δ 11.07 (brs, 1H), 8.86 (d, *J* = 4.0 Hz, 1H), 8.63 (s, 1H), 8.56 (s, 1H), 8.26 (s, 1H), 8.15 (s, 1H), 8.08 - 8.06 (m, 1H), 7.79 - 7.77 (m, 2H), 7.48 - 7.30 (m, 2H), 7.27 (s, 1H), 3.93 (s, 3H), 3.47 (s, 3H), 2.41(s, 3H).

**Preparation of the crystal form E of the compound represented by formula (III)**

100 mg of the compound represented by formula (I) was weighed and added to DMSO (1 mL). The reaction mixture was added with hydrochloric acid (20.51 mg, 208.12 μmol, 17.34 μL, purity: 37%) at 20 to 30°C for reaction, stirred at 20 to 30°C for 24 h, and filtered. The filter cake was washed with ethyl acetate (1 mL × 2), and spin-dried under reduced pressure at 40 to 50°C to obtain the crystal form E of the compound represented by formula (III).

$^1H$ NMR (400 MHz, DMSO-$d_6$) δ 11.04 (brs, 1H), 8.85 (d, *J* = 4.0 Hz, 1H), 8.63 (s, 1H), 8.49 (s, 1H), 8.29 (s, 1H), 8.08 - 8.06 (m, 2H), 7.77 - 7.72 (m, 2H), 7.49 - 7.34 (m, 2H), 7.26 (s, 1H), 3.94 (s, 3H), 3.46 (s, 3H).

**Preparation of the crystal form F of the compound represented by formula (III)**

20 mg of the compound represented by formula (I) and hydrochloric acid (1 eq) were weighed and added to an HPLC vial respectively, mixed and added with 0.5 mL of EtOH/$H_2O$ (19:1, v/v). The reaction mixture was stirred at room temperature for 4 days, centrifuged, and the solid was transferred to a vacuum dryer at 50°C for 0.5 h to obtain the crystal form F of the compound represented by formula (III).

**Preparation of the crystal form G of the compound represented by formula (III)**

100 mg of the compound represented by formula (I) was weighed and added to DMSO (1 mL). The reaction mixture was added with hydrochloric acid (20.51 mg, 208.12 μmol, 17.34 μL, purity: 37%) at 20 to 30°C for reaction, stirred at 20 to 30°C for 24 h, and filtered. The filter cake was washed with ethyl acetate (1 mL × 2), and spin-dried under reduced pressure at 40 to 50°C to obtain the crystal form G of the compound represented by formula (III).

**Preparation of the crystal form H of the compound represented by formula (IV)**

20 mg of the compound represented by formula (I) and sulfuric acid (1 eq) were weighed and added to an HPLC vial respectively, mixed and added with 0.5 mL of ethanol. The reaction mixture was stirred at room temperature for 4 days, centrifuged, and the solid was transferred to a vacuum dryer at 50°C for 0.5 h to obtain the crystal form H of the compound represented by formula (IV).

**Preparation of the crystal form I of the compound represented by formula (IV)**

20 mg of the compound represented by formula (I) and sulfuric acid (1 eq) were weighed and added to an HPLC vial respectively, mixed and added with 0.5 mL of EtOH/$H_2O$ (19:1, v/v). The reaction mixture was stirred at room temperature for 4 days, centrifuged, and the solid was transferred to a vacuum dryer at 50°C for 0.5 h to obtain the crystal form I of the compound represented by formula (IV).

**Preparation of the crystal form J of the compound represented by formula (IV)**

20 mg of the compound represented by formula (I) and sulfuric acid (1 eq) were weighed and added to an HPLC vial respectively, mixed and added with 0.5 mL of tetrahydrofuran. The reaction mixture was stirred at room temperature for 4 days, centrifuged, and the solid was transferred to a vacuum dryer at 50°C for 0.5 h to obtain the crystal form J of the compound represented by formula (IV).

**Preparation of the crystal form K of the compound represented by formula (IV)**

100 mg of the compound represented by formula (I) was weighed and added to DMSO (1 mL). The reaction mixture was added with sulfuric acid (20.41 mg, 208.12 μmol, 11.09 μL, 1 eq) at 20 to 30°C for reaction, stirred at 20 to 30°C for 20 h, added with 2 mL of ethyl acetate, stirred continuously for 20 h, and filtered. The filter cake was washed with ethyl acetate (1 mL × 2), and spin-dried under reduced pressure at 40 to 50°C to obtain the crystal form K of the compound represented by formula (IV).

$^1H$ NMR (400 MHz, DMSO-$d_6$) δ 11.05 (brs, 1H), 8.85 (d, *J* = 4.0 Hz, 1H), 8.63 (s, 1H), 8.53 (s, 1H), 8.30 (s, 1H), 8.09 - 8.06 (m, 2H), 7.77 - 7.75 (m, 2H), 7.49 - 7.33 (m, 2H), 7.24 (s, 1H), 3.94 (s, 3H), 3.46 (s, 3H).

**Preparation of the crystal form L of the compound represented by formula (V)**

20 mg of the compound represented by formula (I) and phosphoric acid (1 eq) were weighed and added to an HPLC vial respectively, mixed and added with 0.5 mL of ethanol. The reaction mixture was stirred at room temperature for 4 days, centrifuged, and the solid was transferred to a vacuum dryer at 50°C for 0.5 h to obtain the crystal form L of the compound represented by formula (V).

$^1H$ NMR (400 MHz, DMSO-$d_6$) δ 8.84 (d, *J* = 4.0 Hz, 1H), 8.63 (s, 1H), 8.47 (s, 1H), 8.28 (s, 1H), 8.09 - 8.02 (m, 2H), 7.77 - 7.70 (m, 2H), 7.50 - 7.45 (m, 3H), 7.36 (t, *J* = 4.0 Hz, 1H), 7.24 (s, 1H), 7.11 (d, *J* = 4.0 Hz, 2H), 3.94 (s, 3H), 2.29 (s, 3H).

**Preparation of the crystal form M of the compound represented by formula (V)**

20 mg of the compound represented by formula (I) and phosphoric acid (1 eq) were weighed and added to an HPLC vial respectively, mixed and added with 0.5 mL of EtOH/$H_2O$ (19:1, v/v). The reaction mixture was stirred at room temperature for 4 days, centrifuged, and the solid was transferred to a vacuum dryer at 50°C for 0.5 h to obtain the crystal form M of the compound represented by formula (V).

**Preparation of the crystal form N of the compound represented by formula (V)**

20 mg of the compound represented by formula (I) and phosphoric acid (1 eq) were weighed and added to an HPLC vial respectively, mixed and added with 0.5 mL of tetrahydrofuran. The reaction mixture was stirred at room temperature for 4 days, centrifuged, and the solid was transferred to a vacuum dryer at 50°C for 0.5 h to obtain the crystal form N of the compound represented by formula (V).

**Preparation of the crystal form O of the compound represented by formula (VI)**

20 mg of the compound represented by formula (I) and p-toluenesulfonic acid (1 eq) were weighed and added to an HPLC vial respectively, mixed and added with 0.5 mL of acetone. The reaction mixture was stirred at room temperature for 4 days, centrifuged, and the solid was transferred to a vacuum dryer at 50°C for 0.5 h to obtain the crystal form O of the compound represented by formula (VI).

**Preparation of the crystal form P of the compound represented by formula (VI)**

20 mg of the compound represented by formula (I) and p-toluenesulfonic acid (1 eq) were weighed and added to an HPLC vial respectively, mixed and added with 0.5 mL of tetrahydrofuran. The reaction mixture was stirred at room temperature for 4 days, centrifuged, and the solid was transferred to a vacuum dryer at 50°C for 0.5 h to obtain the crystal form P of the compound represented by formula (VI).

**Preparation of the crystal form Q of the compound represented by formula (VII)**

20 mg of the compound represented by formula (I) and oxalic acid (1 eq) were weighed and added to an HPLC vial respectively, mixed and added with 0.5 mL of EtOH/$H_2O$ (19:1, v/v). The reaction mixture was stirred at room temperature for 4 days, centrifuged, and the solid was transferred to a vacuum dryer at 50°C for 0.5 h to obtain the crystal form Q of the compound represented by formula (VII).

**Preparation of the crystal form R of the compound represented by formula (VIII)**

20 mg of the compound represented by formula (I) and maleic acid (1 eq) were weighed and added to an HPLC vial respectively, mixed and added with 0.5 mL of acetone. The reaction mixture was stirred at room temperature for 4 days, centrifuged, and the solid was transferred to a vacuum dryer at 50°C for 0.5 h to obtain the crystal form R of the compound represented by formula (VIII).

$^1$H NMR (400 MHz, DMSO-$d_6$) $\delta$ 8.73 (d, *J* = 4.0 Hz, 1H), 8.47 (s, 1H), 8.21 (s, 2H), 8.09 - 8.03 (m, 2H), 7.99 (s, 1H), 7.75 (s, 1H), 7.49 - 7.30 (m, 3H), 7.23 (s, 1H), 6.22 (s, 2H), 3.92 (s, 3H), 3.46 (s, 3H).

**Preparation of the crystal form S of the compound represented by formula (X)**

20 mg of the compound represented by formula (I) and tartaric acid (1 eq) were weighed and added to an HPLC vial respectively, mixed and added with 0.5 mL of acetone. The reaction mixture was stirred at room temperature for 4 days, centrifuged, and the solid was transferred to a vacuum dryer at 50°C for 0.5 h to obtain the crystal form S of the compound represented by formula (X).

$^1$H NMR (400 MHz, DMSO-$d_6$) $\delta$ 8.68 (d, *J* = 4.0 Hz, 1H), 8.47 (s, 1H), 8.13 (s, 1H), 8.09 - 8.02 (m, 2H), 7.95 (s, 1H), 7.77 (s, 1H), 7.49 - 7.33 (m, 3H), 7.24 (s, 1H), 4.32 (s, 3H), 3.94 (s, 3H).

**Embodiment 3: Study on hygroscopicity of the crystal form A of the compound represented by formula (II)**

Experimental materials:

SMS Intrinsic Dynamic Vapor Sorption Analyzer

Experimental methods:

10 to 30 mg of the crystal form A of the compound represented by formula (II) was taken and placed in a DVS sample dish for testing.

Experimental results:

The DVS pattern of the crystal form A of the compound represented by formula (II) is shown in FIG. 35 with ΔW = 1.708%.

Experimental conclusion:

The crystal form A of the compound represented by formula (II) has a hygroscopic weight gain of 1.708% at 25°C and 80% RH, showing its hygroscopicity.

**Embodiment 4: Stability data of the crystal form A of the compound represented by formula (II)**

For tests under high temperature, high humidity and light conditions, samples were put into an open clean weighing bottle, spread into a thin layer of 5 mm or less, and 3 samples (1.1 g/sample) were weighed in parallel at each condition time point. The prepared samples were placed under each condition, and sampled for analysis at each time point.

For accelerated stability and long-term stability tests, samples were packed into a double-layer pharmaceutical low-density polyethylene bag. Each layer of the pharmaceutical low-density polyethylene bag was sealed with buckles, and then the double-layer low-density polyethylene bag was heat-sealed in an aluminum foil bag. 6 samples (1.1 g/sample) were weighed in parallel at each condition time point. The prepared samples were placed under each condition, and sampled for analysis at each time point.

The crystal form A of the compound represented by formula (II) was investigated under the following conditions and sampled at different time points to detect physical properties, and analyzed by HPLC for content and total impurities. The study conditions and detection items are shown in Table 21 below.

**Table 21: Stability test of the crystal form A of the compound represented by formula (II)**

| Test conditions | Time points | Content (%) | Total impurities (%) | Crystal form (XRPD) |
|---|---|---|---|---|
| - | Day 0 | 97.9 | 1.2 | Crystal form A |
| High temperature (60°C, open) | Day 5 | 97.0 | 1.3 | Crystal form A |
| | Day 10 | 98.5 | 1.1 | Crystal form A |
| | Day 30 | 98.2 | 1.2 | Crystal form A |
| High humidity (25°C/relative humidity: 92.5%, open) | Day 5 | 96.8 | 1.2 | Crystal form A |
| | Day 10 | 98.8 | 1.2 | Crystal form A |
| | Day 30 | 98.0 | 1.3 | Crystal form A |
| Light | Day 5 | 98.4 | 1.2 | Crystal form A |
| (Total illumination: $1.2\times10^6$ Lux•hr/NUV: 200 w•hr/$m^2$, open) | Day 10 | 97.7 | 1.1 | Crystal form A |
| Accelerated stability test (40°C/relative humidity: 75%) | Day 0 | 97.9 | 1.2 | Crystal form A |
| | Month 1 | 98.0 | 1.3 | Crystal form A |
| | Month 2 | 97.6 | 1.2 | Crystal form A |
| | Month 3 | 98.0 | 1.2 | Crystal form A |
| | Month 6 | 98.5 | 1.2 | Crystal form A |
| Long-term stability test (25°C/relative humidity: 60%) | Day 0 | 97.9 | 1.2 | Crystal form A |
| | Month 3 | 98.3 | 1.2 | Crystal form A |
| | Month 6 | 98.6 | 1.2 | Crystal form A |
| | Month 9 | 97.6 | 1.2 | Crystal form A |

Experimental conclusion: the crystal form A of the compound represented by formula (II) has good stability.

Bioassay Data:

**Experimental embodiment 1: Study on the inhibitory effect of the crystal form A of the compound represented by formula (II) on cell proliferation**

Experimental purpose:

The compounds of the present disclosure can target and inhibit FGFR and VEGFR pathways, and inhibit tumor cell growth by inhibiting VEGF/VEGFR and FGF/FGFR signaling pathways. In this experiment, human non-small cell lung cancer cells NCI-H1581 with high expression of FGFR1, gastric cancer cells SNU-16 with high expression of FGFR2, and human bladder cancer cells RT112/84 with high expression of FGFR3 were selected. In this experiment, the inhibitory effect of compounds on cell proliferation was investigated by detecting the effect of the compounds of the present disclosure on the cell viability *in vitro* in the tumor cell lines NCI-H1581, SNU-16 and RT-112/84.

Experimental methods and procedures:

Cell culture

The tumor cell lines were cultured in a 37°C, 5% $CO_2$ incubator according to the culture conditions shown in Table 22. Regular passage, and cells in logarithmic growth phase were taken for plating.

Table 22: Cell lines and culture methods

| Cell line | Tumor type | Top of the form |
|---|---|---|
| | | Culture method bottom of the form |
| NCI-H1581 | Lung cancer | RPMI 1640 + 10% FBS |
| SNU-16 | Gastric cancer | RPMI 1640 + 10% FBS |
| RT-112/84 | Bladder cancer | EMEM + 2 mM Glutamine + 1% Non Essential Amino Acids + 10% FBS |

Cell plating

(1) Cells were stained with trypan blue and viable cells were counted.
(2) The cell concentration was adjusted to an appropriate concentration.

| Cell line | Density (per well) |
|---|---|
| NCI-H1581 | 4000 |
| SNU-16 | 7000 |
| RT-112/84 | 8000 |

(3) 90 μL of cell suspension per well was added to a culture plate, and cell-free culture solution was added to the blank control well.
(4) The plate was cultured overnight in a 37°C, 5% $CO_2$ incubator with 100% relative humidity.

Preparation of 10X compound working solution and compound treatment of cells

(1) Preparation of 10X compound working solution: 78 μL of cell culture medium was added to a V-bottom 96-well plate, and 2 μL of compound was pipetted from a 400X compound storage plate and added to the cell culture medium of the 96-well plate. 2 μL of DMSO was added to vehicle control and blank control. After the compound or DMSO were added, it was blown with a pipette and mixed well.
(2) Dosing: 10 μL of 10X compound working solution was added to the cell culture plate. 10 μL of DMSO-cell culture medium mixture was added to vehicle control and blank control. The final concentration of DMSO was 0.25%.

(3) The 96-well plate was returned to the incubator and cultured for 3 days before detection.

CellTiter-Glo luminescent cell viability assay

The following steps were performed according to the instructions of Promega CellTiter-Glo Luminescent Cell Viability Assay Kit (Promega-G7573).

(1) CellTiter-Glo buffer was dissolved and brought to room temperature.
(2) CellTiter-Glo substrate was brought to room temperature.
(3) CellTiter-Glo working solution was prepared by adding CellTiter-Glo buffer to a vial of CellTiter-Glo substrate to dissolve the substrate.
(4) The solution was slowly vortexed and shaken to be fully dissolved.
(5) The cell culture plate was removed and left for 30 min to equilibrate to room temperature.
(6) 50 μL of CellTiter-Glo working solution was added per well (equal to half the volume of cell culture medium per well). The cell plate was wrapped in aluminum foil to be protected from light.
(7) The plate was shaken on an orbital shaker for 2 min to induce cell lysis.
(8) The plate was left at room temperature for 10 min to stabilize the luminescent signal.
(9) The luminescent signal was detected on a 2104 EnVision plate reader.

Data analysis:

The inhibition rate (IR) of the test compound was calculated using the following formula: IR (%) = (1- (RLU compound - RLU blank control) / (RLU vehicle control - RLU blank control)) × 100%. The inhibition rates of compounds at different concentrations were calculated in Excel, and then GraphPad Prism software was used to plot inhibition curves and calculate relevant parameters, including minimum inhibition rate, maximum inhibition rate and $IC_{50}$. The $IC_{50}$ was calculated using the following formula.

$$Y = \text{Minimum inhibition rate} + (\text{Maximum inhibition rate} - \text{Minimum inhibition rate}) / (1 + 10^{\wedge}((\text{LogIC}_{50} - X) \times \text{HillSlope}))$$

X: log(concentration)
Y: response value, which is negatively correlated with X
HillSlope: slope factor
Experimental results: see Table 23.

**Table 23: Study on the inhibitory effect of the crystal form A of the compound represented by formula (II) on cell proliferation**

| | **Tumor cell line** | NCI-H1581 | SNU-16 | RT-112/84 |
|---|---|---|---|---|
| $IC_{50}$ (nM) | **Crystal form A of the compound represented by formula (II)** | 116.7 | 44.5 | 166.8 |

Experimental conclusion: the crystal form A of the compound represented by formula (II) exhibits certain anti-proliferation activity on three tumor cell lines with high expression of FGFR.

**Experimental embodiment 2: *In vitro* activity of the crystal form A of the compound represented by formula (II) on BaF3 cell line**

Experimental purpose:

In this experiment, VEGFR-expressing engineered cells (Ba/F3-TEL-FLT1 (VEGFR1), Ba/F3-TEL-FLT4 (VEGFR3) and Ba/F3-TEL-VEGFR2) were selected to evaluate the inhibitory effect of the crystal form A of the compound represented by formula (II) on *in vitro* proliferation of BaF3 cell line.

Experimental methods and procedures:

Preparation of 1000× solution of crystal form A of the compound represented by formula (II)

A 10 mM mother solution was prepared by dissolving the crystal form A of the compound represented by formula (II) in DMSO. The mother solution was prepared into a total of 9 concentration gradients (10.0000 mM, 2.5000 mM, 0.6250 mM, 0.1563 mM, 0.0391 mM, 0.0098 mM, 0.0024 mM, 0.0006 mM and 0.0002 mM) in a 4-fold dilution and stored in a 96-well plate (Beaver, Suzhou), while the same volume of DMSO solvent was used as a negative control.
1. Cell suspension in logarithmic growth phase was taken and inoculated in a white 96-well cell culture plate (Corning 3917, NY, USA) with a volume of 95 μL per well (2000 cells/well).
2. Diluted medium-compound mixture was added in a volume of 5 μL per well, respectively, and the final concentration of DMSO in Ba/F3-TEL-FLT1 (VEGFR1), Ba/F3-TEL-FLT4 (VEGFR3) and Ba/F3-TEL-VEGFR2 cells was 0.1%.

**Table 24: Cells and culture conditions**

| Cells | Media | Culture conditions |
|---|---|---|
| Ba/F3-TEL-FLT1 (VEGFR1) | RPMI 1640+10% FBS+1% PS+IL-3 | 37°C, 5% $CO_2$ culture |
| Ba/F3-TEL-FLT4 (VEGFR3) | RPMI 1640+10% FBS+1% PS | 37°C, 5% $CO_2$ culture |
| Ba/F3-TEL-VEGFR2 | RPMI 1640+10% FBS+1% PS | 37°C, 5% $CO_2$ culture |

3. The plate was incubated in a 37°C, 5% $CO_2$ incubator for 72 h.
4. The proliferation inhibitory activity and data analysis of the compound by CellTiter-Glo method were the same as in Experimental embodiment 1.

**Experimental results:** see Table 25.

**Table 25: *In vitro* activity of the crystal form A of the compound represented by formula (II) on BaF3 cell line**

| Cell lines | Maximum inhibition (%) | Minimum inhibition (%) | Relative $IC_{50}$ (nM) |
|---|---|---|---|
| **Ba/F3-TEL-FLT1 (VEGFR1)** | 99.34 | -12.05 | 46 |
| **Ba/F3-TEL-FLT4 (VEGFR3)** | 100 | -2.54 | 395.4 |
| **Ba/F3-TEL-VEGFR2** | 99.99 | 8.23 | ~ 9.7 |

Experimental conclusion: the crystal form A of the compound represented by formula (II) exhibits certain anti-proliferation activity on VEGFR-expressing engineered cells (Ba/F3-TEL-FLT1 (VEGFR1), Ba/F3-TEL-FLT4 (VEGFR3) and Ba/F3-TEL-VEGFR2), with strong anti-proliferation activity on Ba/F3-TEL-VEGFR2.

**Experimental embodiment 3: Pharmacokinetic evaluation of the crystal form A of the compound represented by formula (II)**

Experimental purpose: to evaluate the oral absorption of the crystal form A of the compound represented by formula (II) in animals after a single intravenous injection and intragastric administration in SD rats.

Experimental materials: SD rats, EDTA-K2

Experimental operation:

Experimental procedure: 5% DMSO/10% Solutol/85% water was used as a vehicle, and a clarified solution of the crystal form A of the compound represented by formula (II) at a concentration of 5 mg/mL was intravenously injected into male and female SD rats (overnight fasting, 7 to 11 weeks old) at a dose of 10 mg/kg.
The crystal form A of the compound represented by formula (II) of 5% DMSO/10% Kolliphor HS15/85% (0.2% (v/v) Tween80 aqueous solution) at 1 mg/mL, 3 mg/mL and 10 mg/mL was administered by gavage to male and female SD rats (overnight fasting, 7 to 11 weeks old) at doses of 10 mg/kg, 30 mg/kg and 100 mg/kg. At 0.25, 0.5, 1.0, 2.0, 4.0,

8.0 and 24 h (0.083 h was added in group IV) after administration, approximately 0.2 mL of blood was collected from the jugular vein of the animals in all four groups, placed in an anticoagulant tube added with EDTA-K2, and centrifuged for the separation of plasma. Plasma concentrations were determined by LC-MS/MS method, and relevant pharmacokinetic parameters were calculated using WinNonlin™ Version 6.3 (Pharsight, Mountain View, CA) pharmacokinetic software with non-compartmental model linear-log trapezoidal method.

Experimental results:

After a single intravenous injection of 1 mg/kg of the crystal form A of the compound represented by formula (II) into male and female SD rats, the plasma clearances (Cl) were 8.78 and 7.03 mL/min/kg, respectively, the apparent volumes of distribution at steady state ($Vd_{ss}$) were 0.419 and 0.366 L/kg, respectively, the elimination half-lives ($T_{1/2}$) were 0.675 and 0.765 h, respectively, and the values of area under the plasma concentration-time curve ($AUC_{0-last}$) from time 0 to the last quantifiable time point were 3980 and 4930 nM h, respectively.

There were no significant gender differences in systemic exposure ($AUC_{0-last}$ and $C_0$) between male and female SD rats at intravenous doses.

After a single intragastric administration of 10, 30 and 100 mg/kg of the crystal form A of the compound represented by formula (II) to male SD rats, the peak concentrations ($C_{max}$) of the crystal form A of the compound represented by formula (II) were 3780, 16700 and 19900 nM, respectively, and the peak time ($T_{max}$) appeared at 0.500, 1.00 and 0.833 h after administration, respectively. The $AUC_{0-last}$ were 7150, 38200 and 113000 nM·h, respectively. The drug bioavailability of the intragastric administration group at a dose of 10 mg/kg was 18.0%.

After a single intragastric administration of 10, 30 and 100 mg/kg of the crystal form A of the compound represented by formula (II) to female SD rats, the peak concentrations ($C_{max}$) of the crystal form A of the compound represented by formula (II) were 9170, 24500 and 27400 nM, respectively, and the peak time ($T_{max}$) appeared at 0.667, 1.00 and 0.500 h after administration, respectively. The $AUC_{0-last}$ were 24800, 65600 and 125000 nM·h, respectively. The drug bioavailability of the intragastric administration group at a dose of 10 mg/kg was 50.3%.

There were no significant gender differences in systemic exposure ($AUC_{0-last}$ and $C_{max}$) between male and female SD rats at intragastric doses of 30 and 100 mg/kg.

**Conclusion:** the crystal form A of the compound represented by formula (II) has a low clearance, acceptable oral bioavailability and good druggability in rat species.

**Experimental embodiment 4: Pharmacokinetic study of the compounds of the present disclosure in rats**

Experimental animals:

SD rats (overnight fasting, 7 to 11 weeks old)

Experimental operation:

Experimental procedure: 5% DMSO/10% Solutol/85% water was used as a vehicle, and a clarified solution of the compound of the present disclosure or its crystal form at a concentration of 10 mg/mL was intravenously injected into male SD rats (overnight fasting, 7 to 11 weeks old) at a dose of 100 mg/kg.

The compound of the present disclosure or its crystal form of 5% DMSO/10% Kolliphor HS 15/85% (0.2% (v/v) Tween80 aqueous solution) at 10 mg/mL was administered by gavage to male SD rats (overnight fasting, 7 to 11 weeks old) at a dose of 100 mg/kg. At 0.25, 0.5, 1.0, 2.0, 4.0, 8.0 and 24 h after administration, approximately 0.2 mL of blood was collected from the jugular vein of the animals in all three groups, placed in an anticoagulant tube added with EDTA-K2, and centrifuged for the separation of plasma. Plasma concentrations were determined by LC-MS/MS method, and relevant pharmacokinetic parameters were calculated using WinNonlin™ Version 6.3 (Pharsight, Mountain View, CA) pharmacokinetic software with non-compartmental model linear-log trapezoidal method.

Experimental results:

**Table 26: Results of pharmacokinetic test**

| Dose: 100 mpk | Compound represented by formula (I) | Crystal form E of the compound represented by formula (III) | Crystal form D of the compound represented by formula (II) |
|---|---|---|---|
| $C_{max}$ (nmol/L) | 3755 | 8775 | 22100 |
| $T_{max}$ (h) | 0.500 | 0.500 | 2.50 |
| $T_{1/2}$ (h) | 2.25 | 2.57 | 1.85 |
| $T_{last}$ (h) | 24.0 | 24.0 | 24.0 |
| $AUC_{0\text{-}last}$ (h*nmol/L) | 12595 | 32288 | 178619 |
| $AUC_{0\text{-}inf}$ (h*nmol/L) | 12603 | 32335 | 178663 |

Experimental conclusion: the crystal form D of the compound represented by formula (II) exhibits higher exposure and $C_{max}$ than the crystal form E of the compound represented by formula (III) and the compound represented by formula (I) at the same dose.

**Experimental embodiment 5: Antitumor activity test of the crystal form A of the compound represented by formula (II) on *in vivo* animal tumor model**

**Experimental purpose:**

In this experiment, the antitumor effect of the crystal form A of the compound represented by formula (II) was evaluated using Renca subcutaneous xenograft tumor nude mouse model.

**Experimental animals:**

Female Balb/c mice (6 to 8 weeks old)

**Experimental methods:**

**Cell culture**

Mouse renal carcinoma Renca cells (ATCC-CRL-2947) were adherent cultured *in vitro* under the conditions of RPMI 1640 Medium with 10% fetal bovine serum, 0.1 mM non-essential amino acids, 1 mM sodium pyruvate, 2 mM glutamine, 100 U/mL penicillin and 100 μg/mL streptomycin, and cultured in a 37°C, 5% $CO_2$ incubator. Routine passage was performed twice a week. When the cell saturation density was 80% to 90% and the number of cells reached the required level, the cells were collected, counted, and inoculated.

**Cell inoculation**

0.1 mL ($1 \times 10^5$) Renca cells were subcutaneously inoculated on the dorsal side of the right upper limb of each mouse, and grouping and administration were initiated when the average tumor volume reached approximately 50 to 80 $mm^3$.

**Tumor measurement and experimental indicators**

Tumor diameters were measured twice a week with vernier calipers. The formula for calculating tumor volume was: $V = 0.5a \times b^2$, where *a* and *b* were the long and short diameters of the tumor, respectively.

The antitumor efficacy of compounds was evaluated by TGI (%) or relative tumor proliferation rate T/C (%). Relative tumor proliferation rate T/C (%) = $T_{RTV}$ / $C_{RTV}$ × 100% ($T_{RTV}$: average RTV of the treatment group; $C_{RTV}$: average RTV of the negative control group). Relative tumor volume (RTV) was calculated according to the results of tumor measurement, and the formula was RTV = $V_t$ / $V_0$, where $V_0$ was the tumor volume measured at the time of

grouping and administration (i.e. D0), Vt was the tumor volume of the corresponding mouse in one measurement, and the $T_{RTV}$ and $C_{RTV}$ data were collected on the same day.

TGI (%) reflected the tumor growth inhibition rate. TGI (%) = [(1 - (Average tumor volume at the end of administration in a treatment group - Average tumor volume at the beginning of administration in this treatment group) / (Average tumor volume at the end of treatment in the solvent control group - Average tumor volume at the beginning of treatment in the solvent control group)] × 100%.

Statistical analysis was performed based on the RTV data at the end of the experiment using SPSS software. Comparisons between two groups were performed by T test, and comparisons among three or more groups were performed by one-way ANOVA. If the variance was equal (no significant difference in F value), the Tukey's method was used for analysis, and if the variance was not equal (significant difference in F value), the Games-Howell method was used for testing. $p < 0.05$ was considered a significant difference.

**Experimental results:**

The dose group (100 mg/kg) of the crystal form A of the compound represented by formula (II) had a significant inhibitory effect on tumor growth with a significant difference from the solvent control group, with p = 0.031.

**Table 27: Results of *in vivo* antitumor activity test in mice**

| | Renca xenograft tumor model | TGI% (last administration on day 19) | p-value |
|---|---|---|---|
| Crystal form A of the compound represented by formula (II) | 100 mg/kg QD | 72% | 0.031 |
| Note: QD: administration once a day, TGI%: tumor growth inhibition rate | | | |

Experimental conclusion: the crystal form A of the compound represented by formula (II) exhibits an excellent tumor inhibitory effect in the Renca xenograft tumor model.

## Claims

1. A compound represented by formula (II),

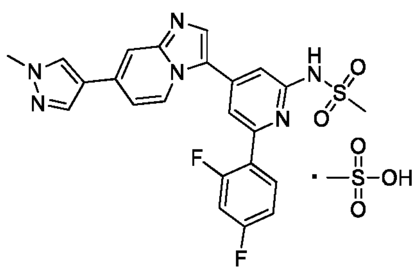

(II)

.

2. A crystal form A of a compound represented by formula (II), wherein the crystal form A has an X-ray powder diffraction pattern comprising characteristic diffraction peaks at the following 2θ angles: 7.65±0.20°, 17.70±0.20°, 24.02±0.20°,

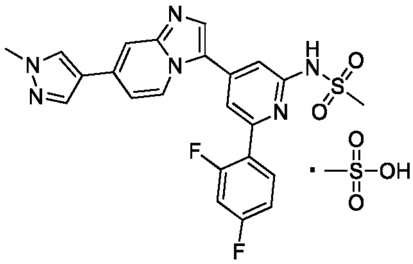

(II)

.

3. The crystal form A according to claim 2, wherein the X-ray powder diffraction pattern of the crystal form A has characteristic diffraction peaks at the following 2θ angles: 7.65±0.20°, 16.84±0.20°, 17.70±0.20°, 20.10±0.20°, 20.91±0.20°, 24.02±0.20°.

4. The crystal form A according to claim 3, wherein the X-ray powder diffraction pattern of the crystal form A has characteristic diffraction peaks at the following 2θ angles: 7.65±0.20°, 16.84±0.20°, 17.70±0.20°, 20.10±0.20°, 20.91±0.20°, 24.02±0.20°, 24.98±0.20°, 26.60±0.20°.

5. The crystal form A according to claim 4, wherein the X-ray powder diffraction pattern of the crystal form A has characteristic diffraction peaks at the following 2θ angles: 7.649°, 12.713°, 16.841°, 17.695°, 20.100°, 20.912°, 24.018°, 24.976°, 26.599°, 28.076°.

6. The crystal form A according to claim 5, wherein the XRPD pattern of the crystal form A is shown in FIG. 1.

7. The crystal form A according to any one of claims 2 to 6, wherein the differential scanning calorimetry curve of the crystal form A has an endothermic peak with a peak value at 283.9±3.0°C.

8. The crystal form A according to claim 7, wherein the DSC pattern of the crystal form A is shown in FIG. 2.

9. The crystal form A according to any one of claims 2 to 6, wherein the thermogravimetric analysis curve of the crystal form A has a weight loss of 0.955% at 200.0±3.0°C.

10. The crystal form A according to claim 9, wherein the TGA pattern of the crystal form A is shown in FIG. 3.

11. A crystal form B of the compound represented by formula (II), wherein the crystal form B has an X-ray powder diffraction pattern comprising characteristic diffraction peaks at the following 2θ angles: 6.75±0.20°, 9.94±0.20°, 23.94±0.20°.

12. The crystal form B according to claim 11, wherein the X-ray powder diffraction pattern of the crystal form B has characteristic diffraction peaks at the following 2θ angles: 6.75±0.20°, 9.94±0.20°, 11.70±0.20°, 17.52±0.20°, 20.36±0.20°, 23.94±0.20°,

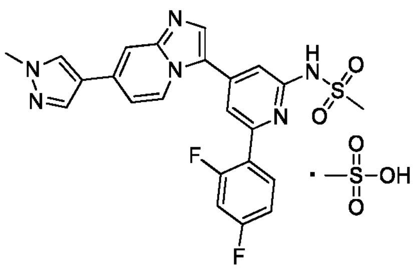

(II)

.

13. The crystal form B according to claim 12, wherein the X-ray powder diffraction pattern of the crystal form B has characteristic diffraction peaks at the following 2θ angles: 6.75±0.20°, 9.94±0.20°, 11.70±0.20°, 14.38±0.20°,

17.52±0.20°, 18.95±0.20°, 20.36±0.20°, 23.94±0.20°.

**14.** The crystal form B according to claim 13, wherein the X-ray powder diffraction pattern of the crystal form B has characteristic diffraction peaks at the following 2θ angles: 6.75°, 9.94°, 11.70°, 13.62°, 14.38°, 15.47°, 17.52°, 18.95°, 20.36°, 23.94°, 25.34°, 25.46°, 26.93°, 28.79°.

**15.** The crystal form B according to claim 14, wherein the XRPD pattern of the crystal form B is shown in FIG. 4.

**16.** The crystal form B according to any one of claims 11 to 15, wherein the differential scanning calorimetry curve of the crystal form B has endothermic peaks with an onset at 57.40±3.0°C and 296.86±3.0°C, respectively.

**17.** The crystal form B according to claim 16, wherein the DSC pattern of the crystal form B is shown in FIG. 5.

**18.** The crystal form B according to any one of claims 11 to 15, wherein the thermogravimetric analysis curve of the crystal form B has a weight loss of 10.53% at 150.0±3.0°C.

**19.** The crystal form B according to claim 18, wherein the TGA pattern of the crystal form B is shown in FIG. 6.

**20.** A crystal form D of a compound represented by formula (II), wherein the crystal form D has an X-ray powder diffraction pattern comprising characteristic diffraction peaks at the following 2θ angles: 10.78±0.20°, 13.64±0.20°, 16.66±0.20°,

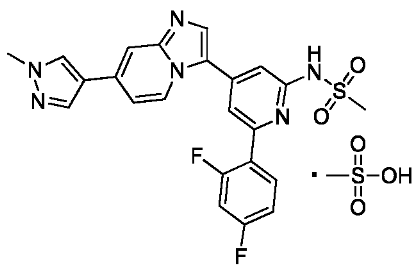

(II)

.

**21.** The crystal form D according to claim 20, wherein the X-ray powder diffraction pattern of the crystal form D has characteristic diffraction peaks at the following 2θ angles: 10.78±0.20°, 13.64±0.20°, 16.66±0.20°, 19.63±0.20°, 21.13±0.20°, 25.40±0.20°.

**22.** The crystal form D according to claim 21, wherein the X-ray powder diffraction pattern of the crystal form D has characteristic diffraction peaks at the following 2θ angles: 5.51±0.20°, 8.73±0.20°, 10.78±0.20°, 13.64±0.20°, 16.66±0.20°, 19.63±0.20°, 21.13±0.20°, 25.40±0.20°.

**23.** The crystal form D according to claim 22, wherein the X-ray powder diffraction pattern of the crystal form D has characteristic diffraction peaks at the following 2θ angles: 5.51°, 8.10°, 8.73°, 10.78°, 12.64°, 13.64°, 14.47°, 14.92°, 15.80°, 16.66°, 17.47°, 19.03°, 19.63°, 21.13°, 21.69°, 22.02°, 22.20°, 23.84°, 24.31°, 25.40°, 25.93°, 26.28°, 26.84°, 27.41°, 27.93°, 29.10°, 30.01°, 30.78°, 32.16°, 32.78°, 33.57°, 38.41°.

**24.** The crystal form D according to claim 23, wherein the XRPD pattern of the crystal form D is shown in FIG. 10.

**25.** The crystal form D according to any one of claims 20 to 24, wherein the differential scanning calorimetry curve of the crystal form D has endothermic peaks with an onset at 27.1±3.0°C and 298.8±3.0°C, respectively.

**26.** The crystal form D according to claim 25, wherein the DSC pattern of the crystal form D is shown in FIG. 11.

**27.** The crystal form D according to any one of claims 20 to 24, wherein the thermogravimetric analysis curve of the crystal form D has a weight loss of 3.15% at 150.0±3.0°C.

**28.** The crystal form D according to claim 27, wherein the TGA pattern of the crystal form D is shown in FIG. 12.

**29.** A use of the compound according to claim 1, or the crystal form A according to any one of claims 2 to 10, the crystal form B according to any one of claims 11 to 19, or the crystal form D according to any one of claims 20 to 28 in the manufacture of a medicament for the treatment of diseases related to FGFR/VEGFR dual kinase inhibitors.

**30.** The use according to claim 29, wherein the medicament related to FGFR/VEGFR dual kinase inhibitors is a medicament for the treatment of solid tumors.

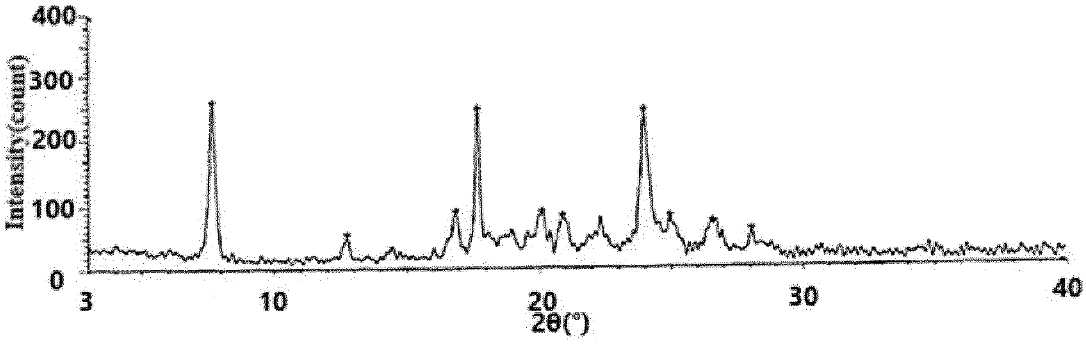
Intensity(count)
400
300
200
100
0
3
10
20
30
40
2θ(°)

FIG. 1

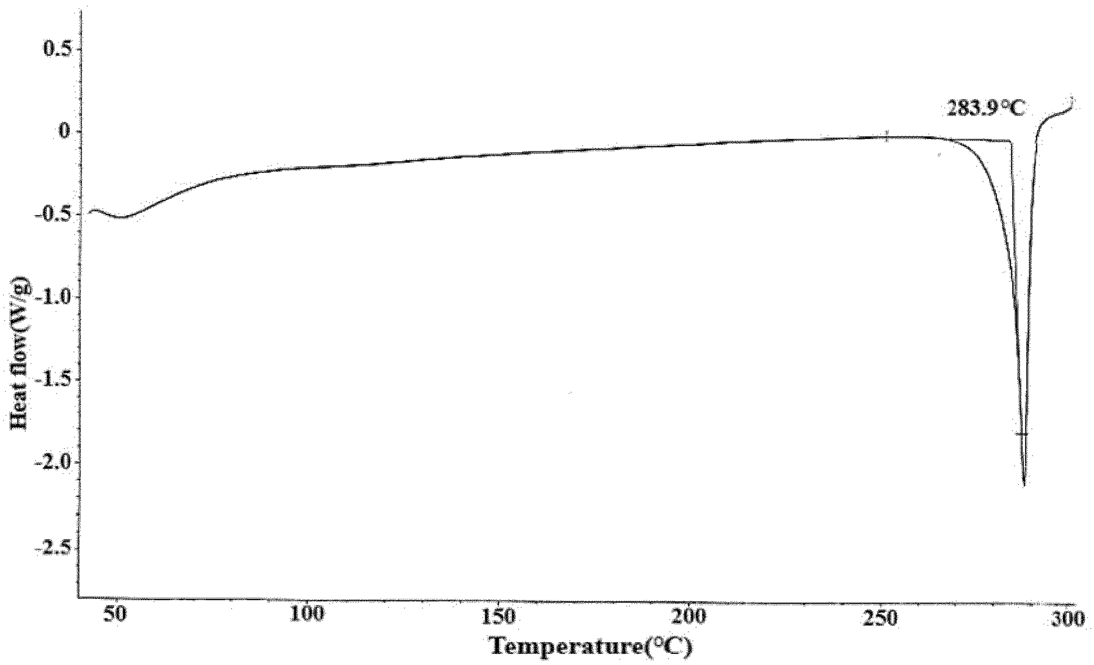
283.9°C
0.5
0
-0.5
-1.0
-1.5
-2.0
-2.5
Heat flow(W/g)
50
100
150
200
250
300
Temperature(°C)

FIG. 2

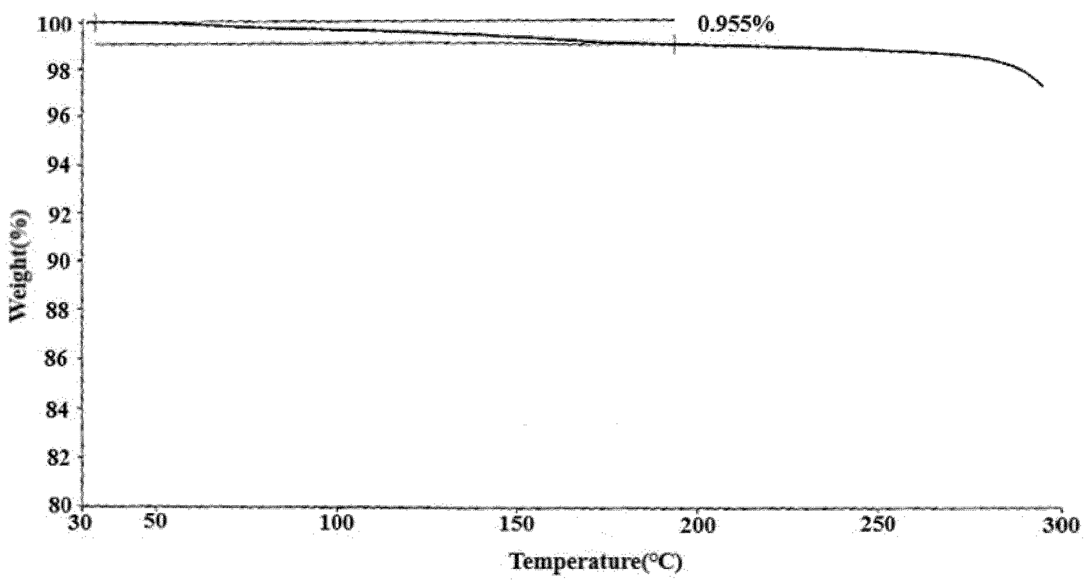
0.955%
Weight(%)
100
98
96
94
92
90
88
86
84
82
80
30
50
100
150
200
250
300
Temperature(°C)

FIG. 3

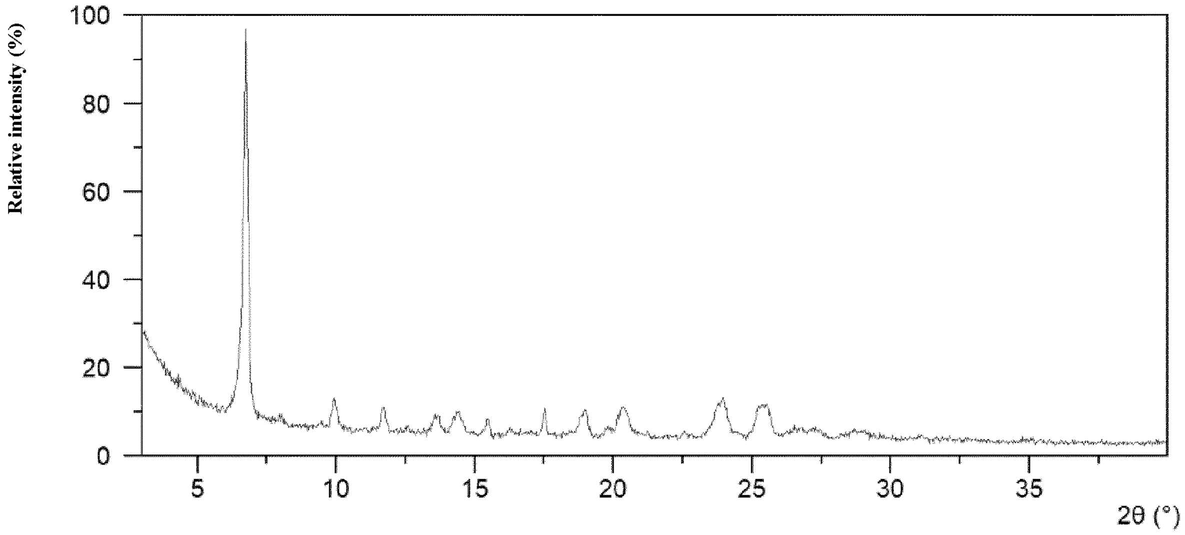
Relative intensity (%)
100
80
60
40
20
0
5
10
15
20
25
30
35
2θ (°)

FIG. 4

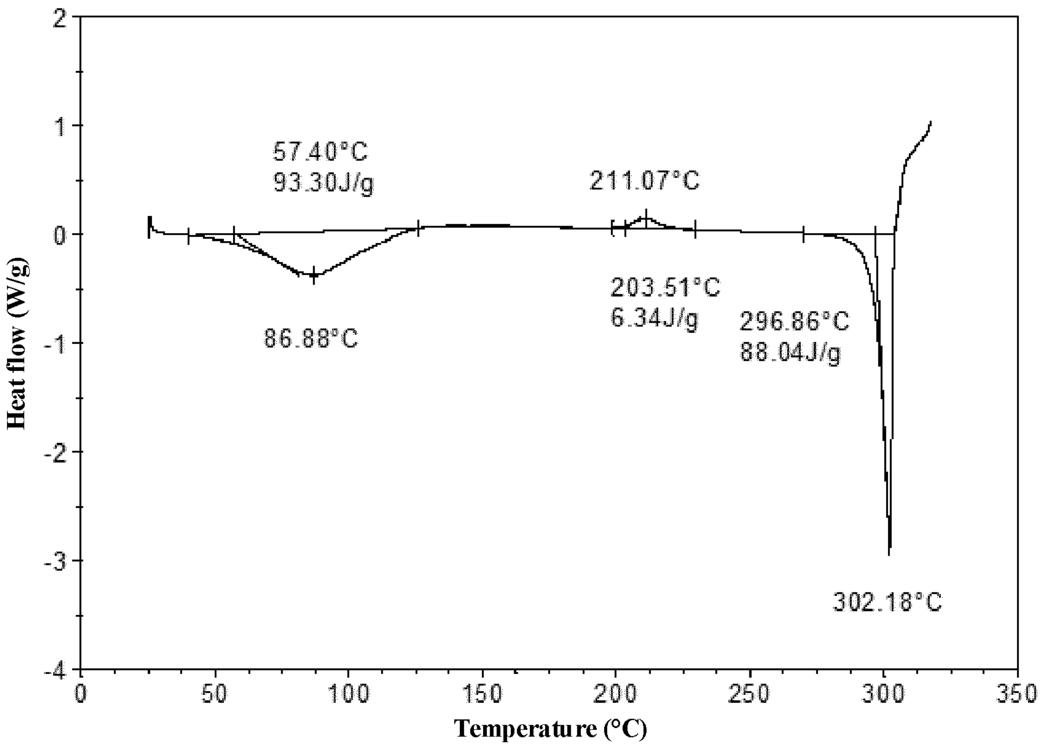
57.40°C
93.30J/g
211.07°C
203.51°C
6.34J/g
296.86°C
88.04J/g
86.88°C
302.18°C
Heat flow (W/g)
Temperature (°C)
2
1
0
-1
-2
-3
-4
0
50
100
150
200
250
300
350

FIG. 5

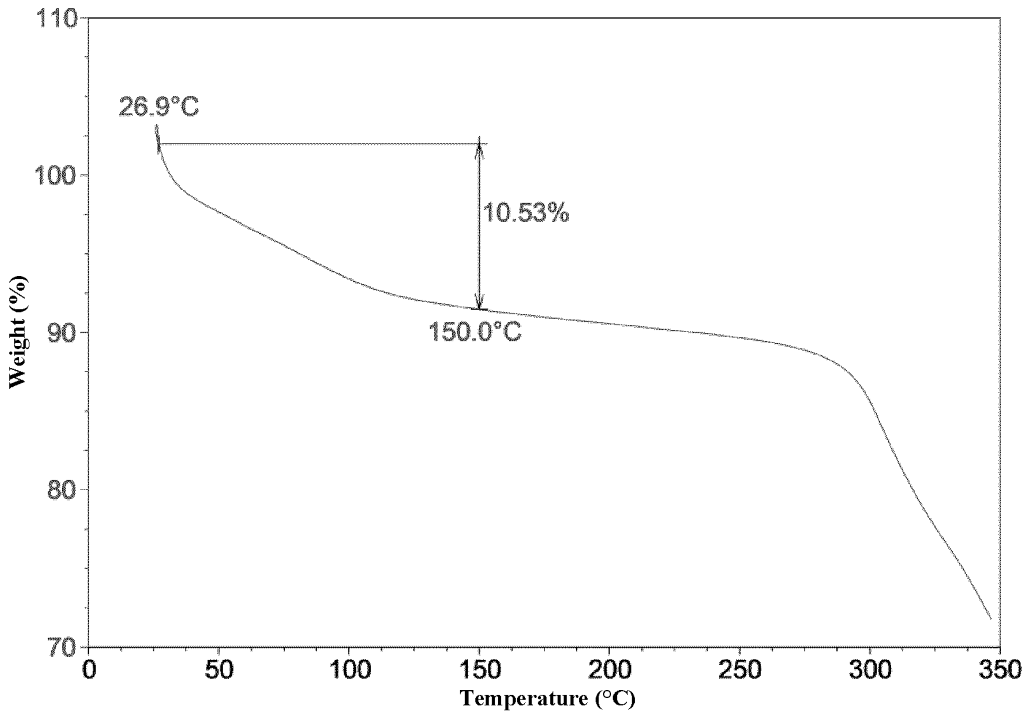
26.9°C
10.53%
150.0°C
Weight (%)
Temperature (°C)
110
100
90
80
70
0
50
100
150
200
250
300
350

FIG. 6

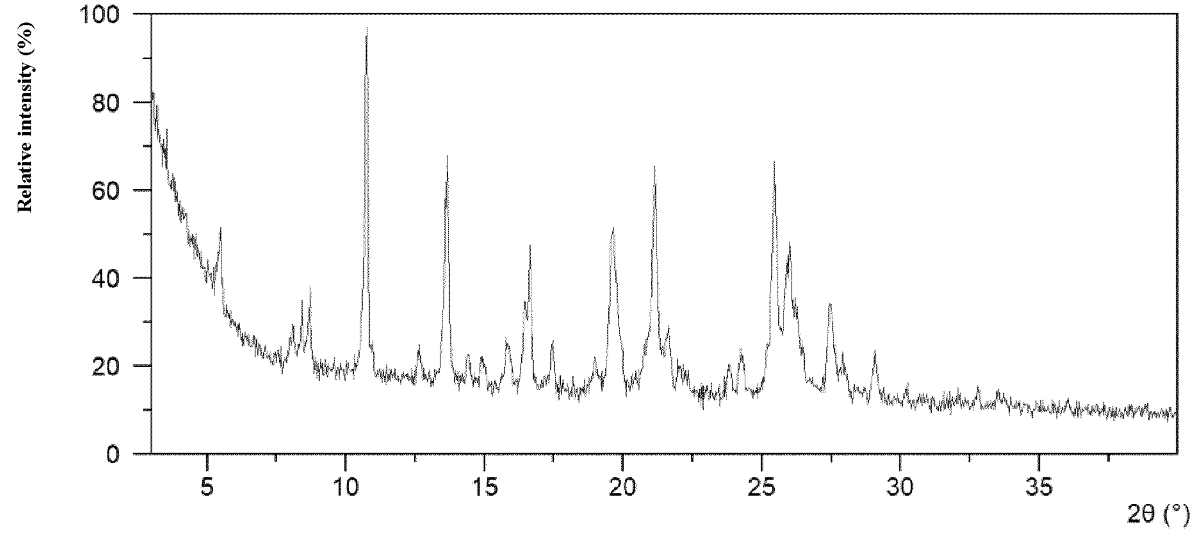
Relative intensity (%)
100
80
60
40
20
0
5
10
15
20
25
30
35
2θ (°)

FIG. 7

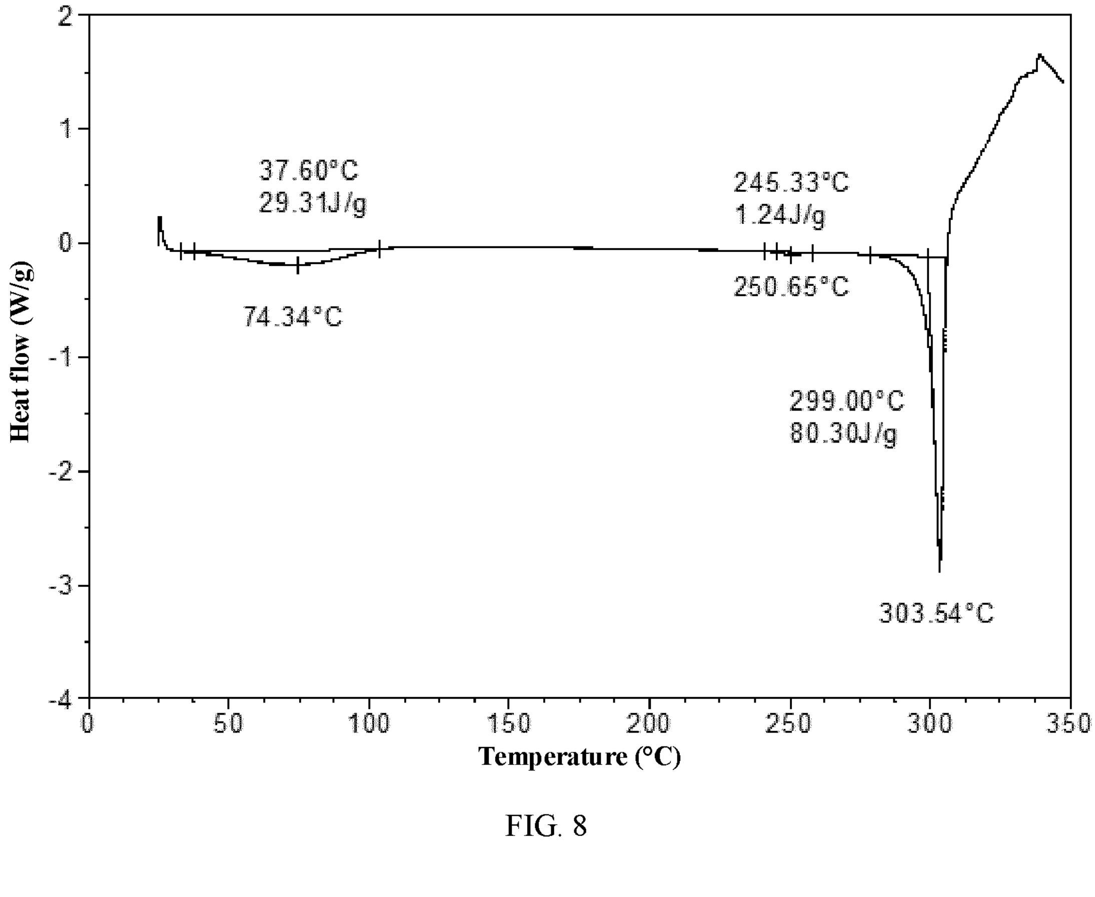
Heat flow (W/g)
Temperature (°C)
37.60°C
29.31J/g
74.34°C
245.33°C
1.24J/g
250.65°C
299.00°C
80.30J/g
303.54°C
2
1
0
-1
-2
-3
-4
0
50
100
150
200
250
300
350

FIG. 8

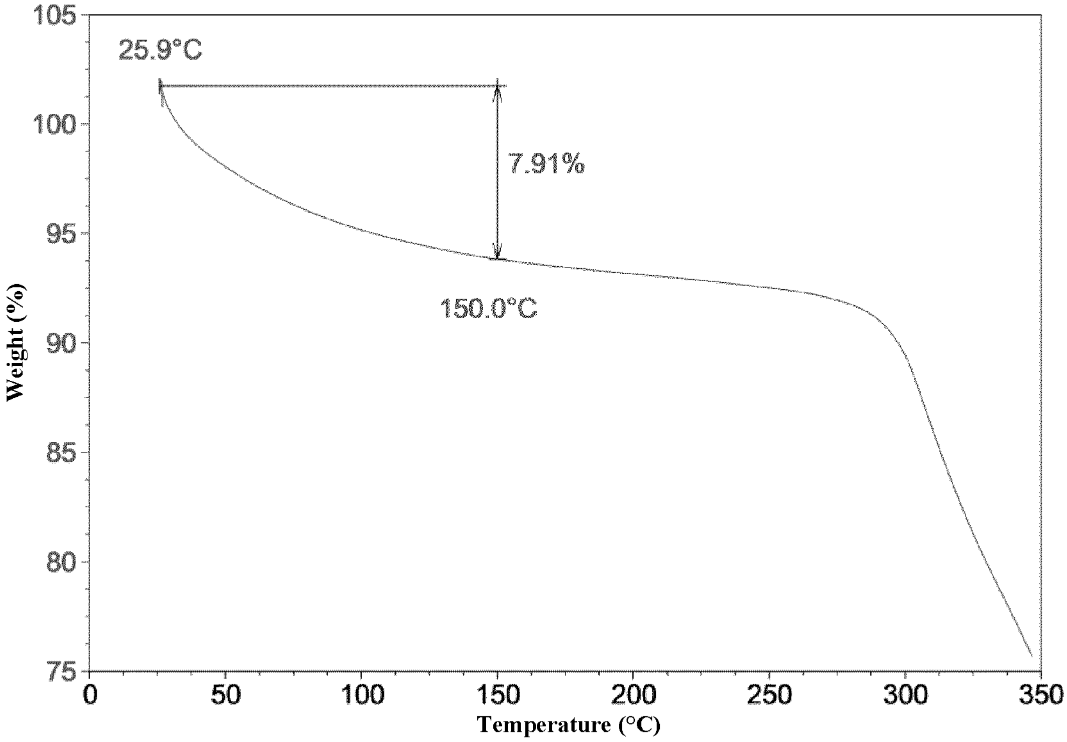

105
100
95
90
85
80
75
25.9°C
7.91%
150.0°C
Weight (%)
0
50
100
150
200
250
300
350
Temperature (°C)


FIG. 9

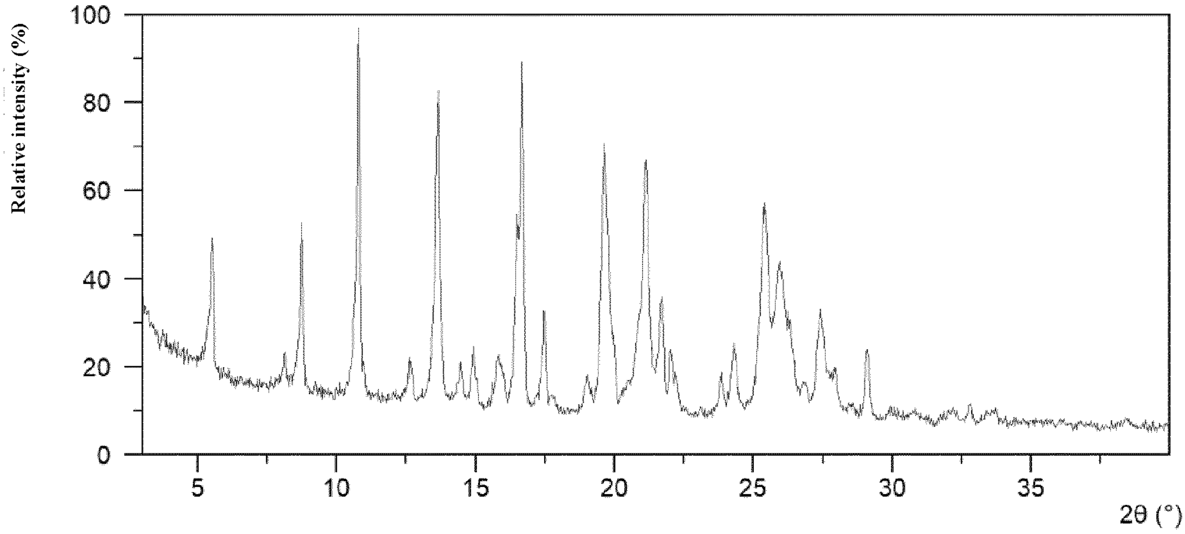

100
80
60
40
20
0
Relative intensity (%)
5
10
15
20
25
30
35
2θ (°)


FIG. 10

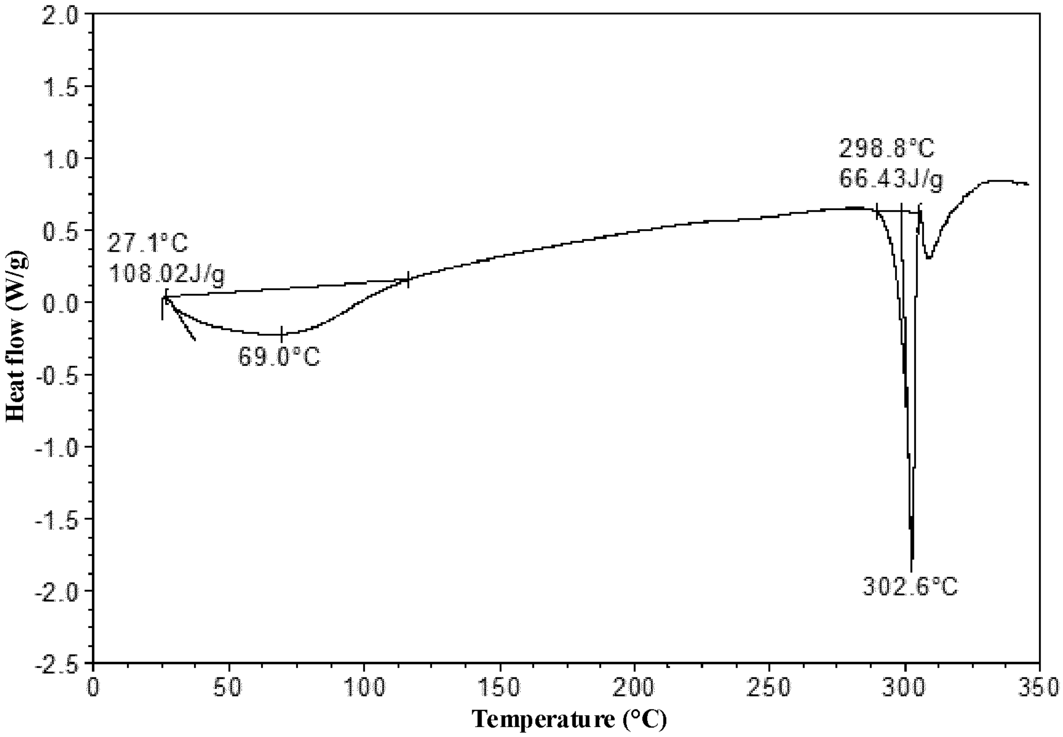
2.0
1.5
1.0
0.5
0.0
-0.5
-1.0
-1.5
-2.0
-2.5
Heat flow (W/g)
27.1°C
108.02J/g
69.0°C
298.8°C
66.43J/g
302.6°C
0
50
100
150
200
250
300
350
Temperature (°C)

FIG. 11

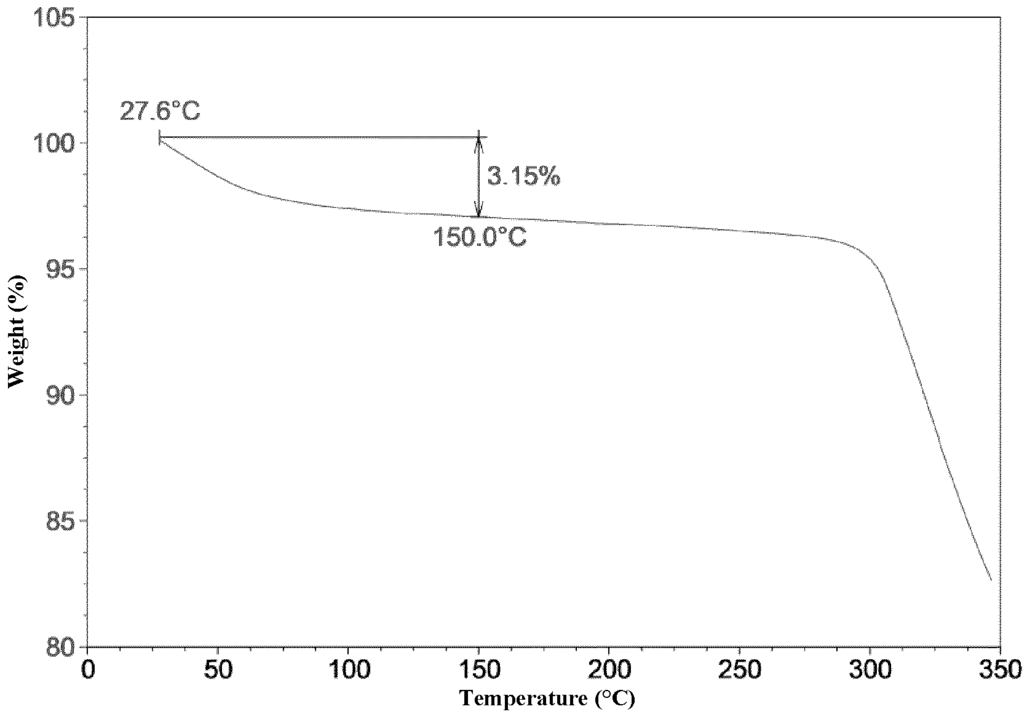
105
100
95
90
85
80
Weight (%)
27.6°C
3.15%
150.0°C
0
50
100
150
200
250
300
350
Temperature (°C)

FIG. 12

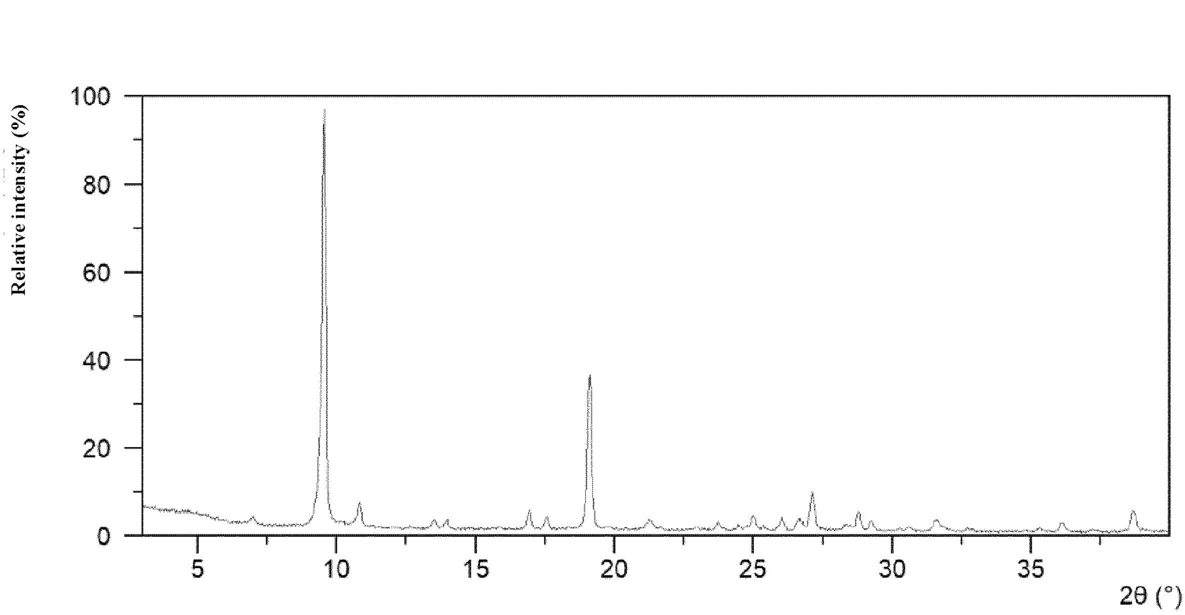
Relative intensity (%)
100
80
60
40
20
0
5
10
15
20
25
30
35
2θ (°)

FIG. 13

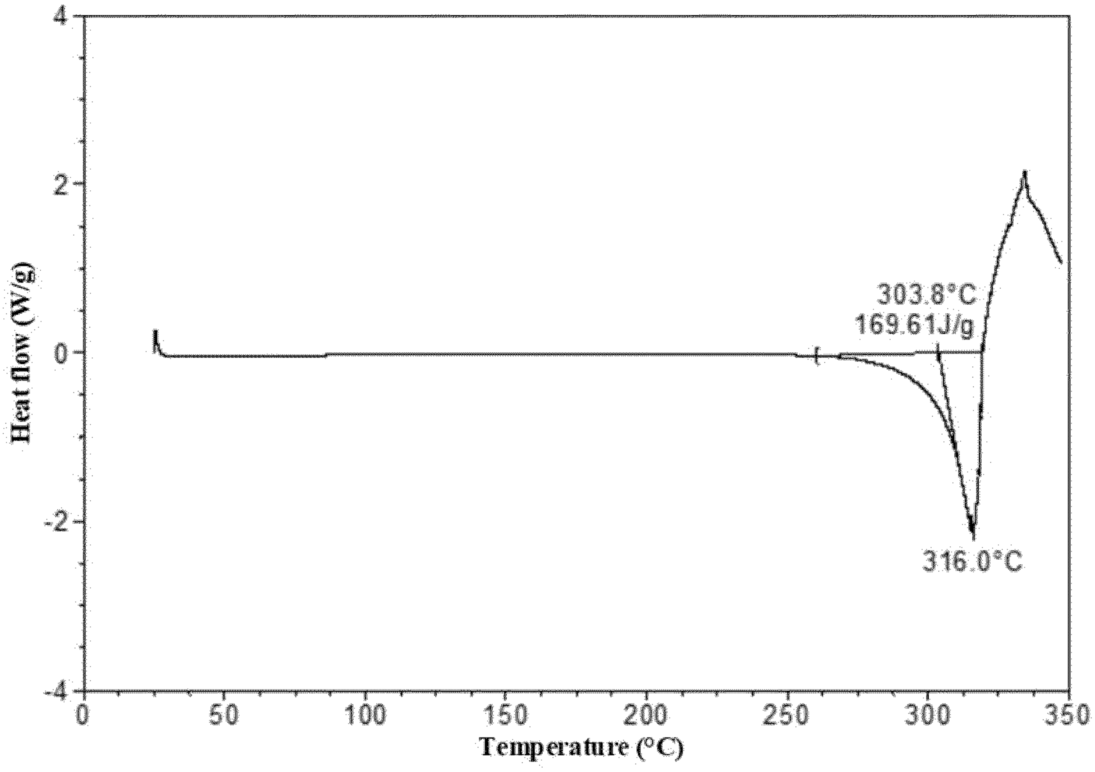
Heat flow (W/g)
4
2
0
-2
-4
0
50
100
150
200
250
300
350
Temperature (°C)
303.8°C
169.61J/g
316.0°C

FIG. 14

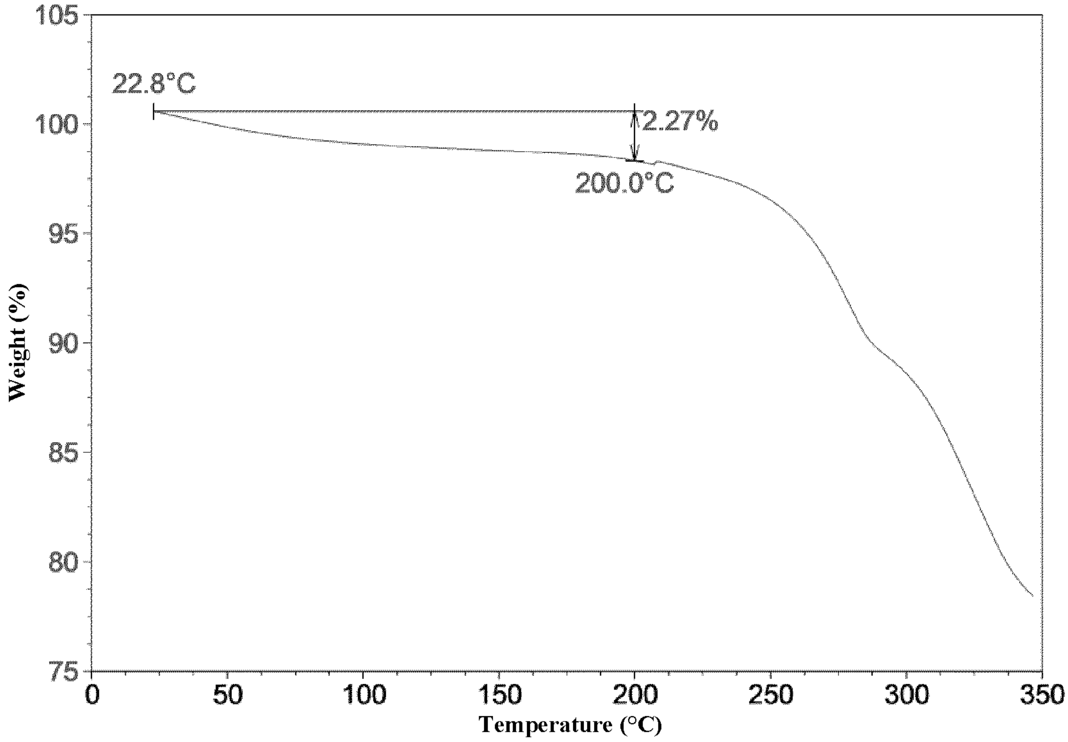
22.8°C
2.27%
200.0°C
Weight (%)
Temperature (°C)
105
100
95
90
85
80
75
0
50
100
150
200
250
300
350

FIG. 15

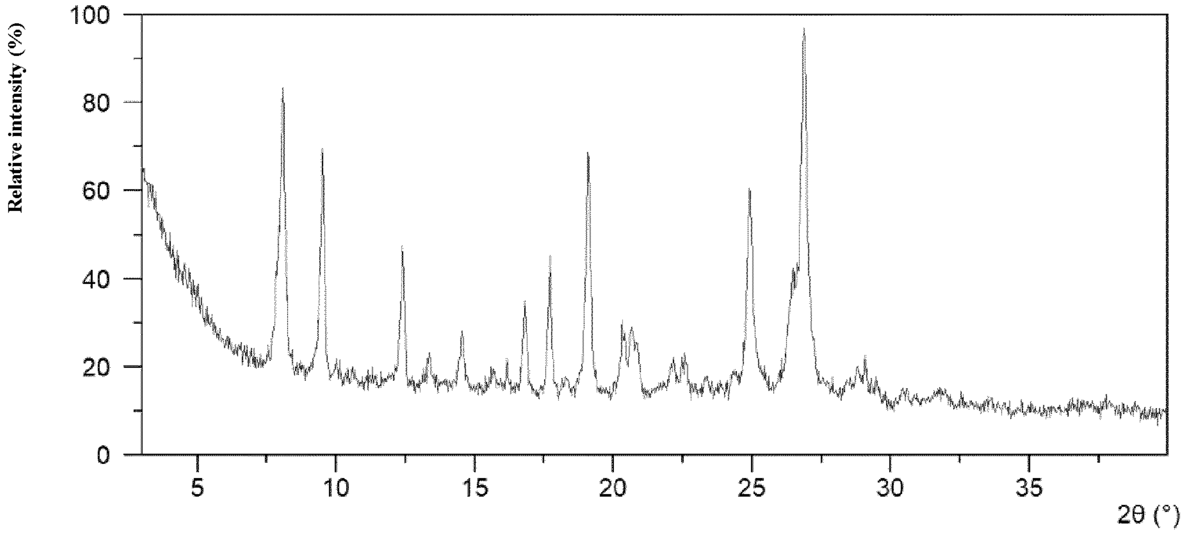
Relative intensity (%)
2θ (°)
100
80
60
40
20
0
5
10
15
20
25
30
35

FIG. 16

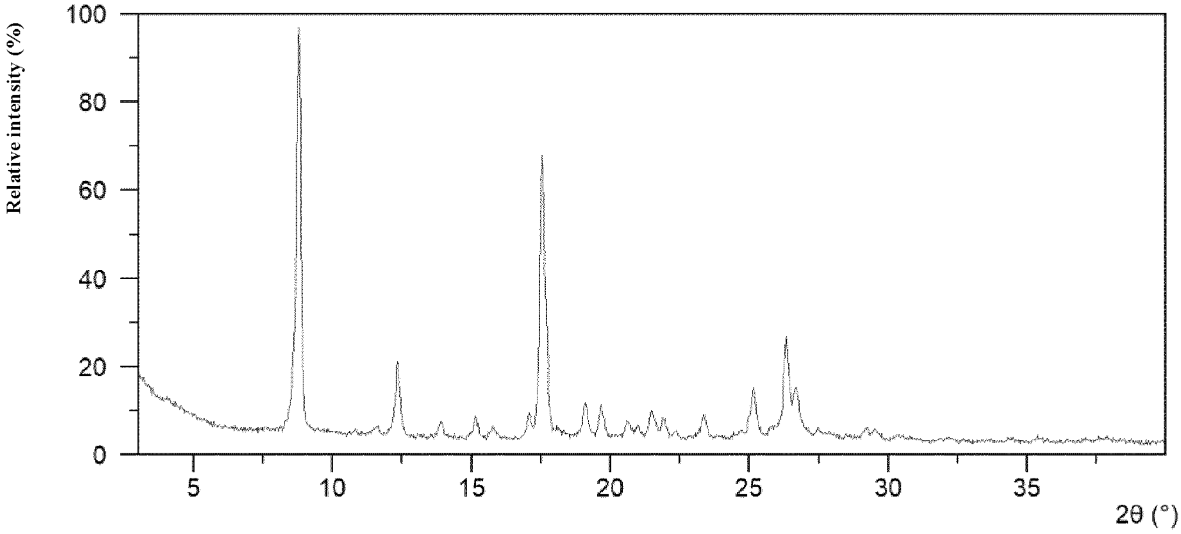
Relative intensity (%)
100
80
60
40
20
0
5
10
15
20
25
30
35
2θ (°)

FIG. 17

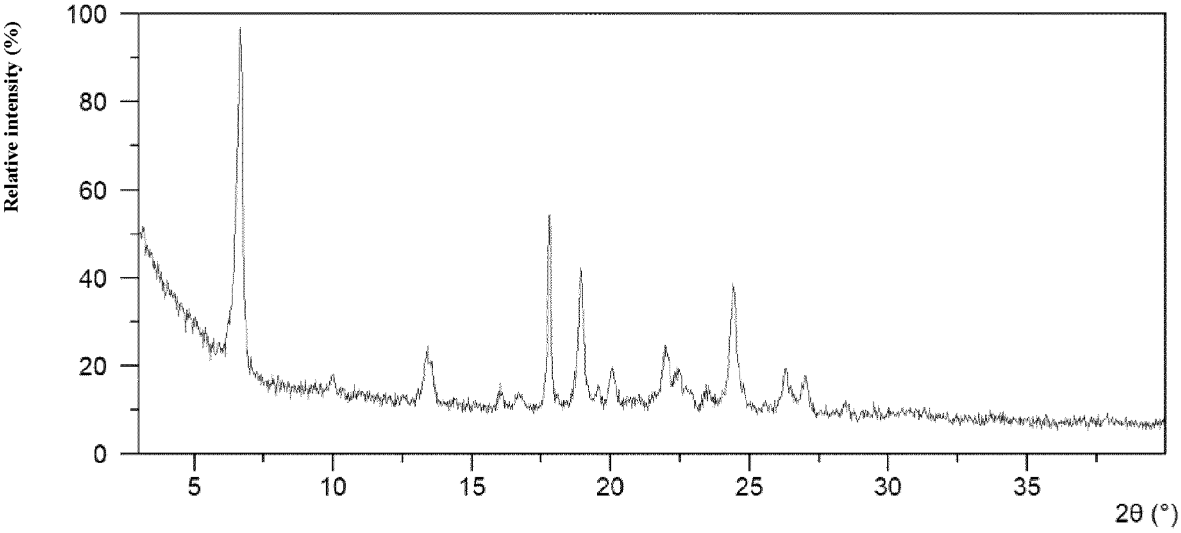
Relative intensity (%)
100
80
60
40
20
0
5
10
15
20
25
30
35
2θ (°)

FIG. 18

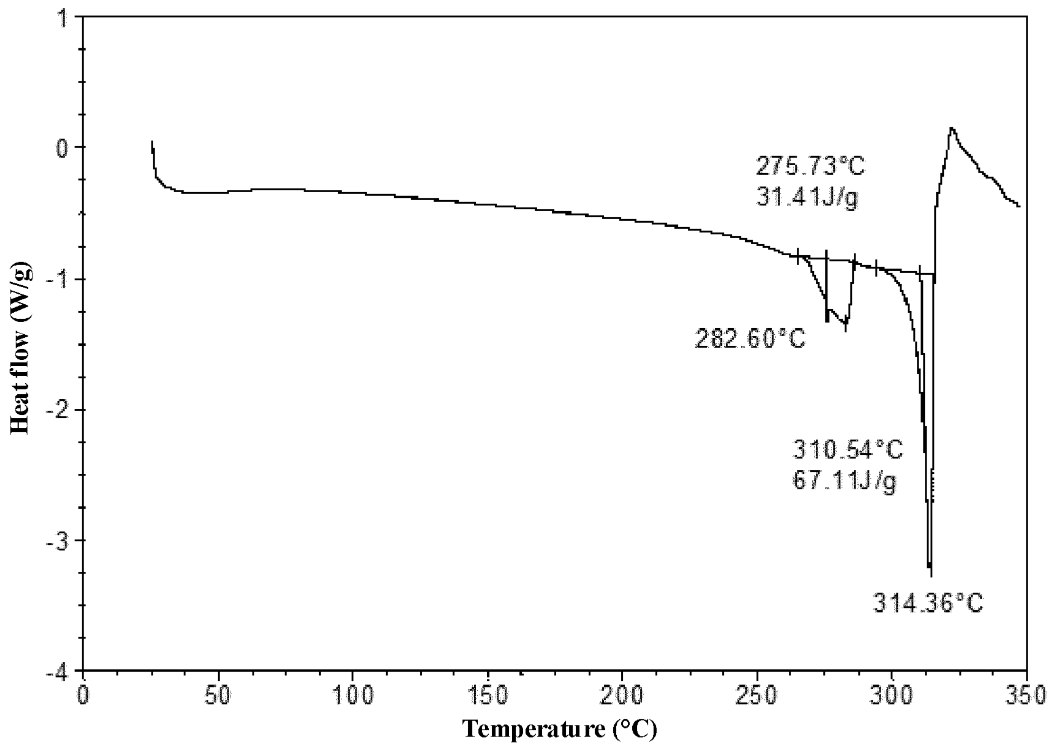

Heat flow (W/g)
1
0
-1
-2
-3
-4
0
50
100
150
200
250
300
350
Temperature (°C)
275.73°C
31.41J/g
282.60°C
310.54°C
67.11J/g
314.36°C


FIG. 19

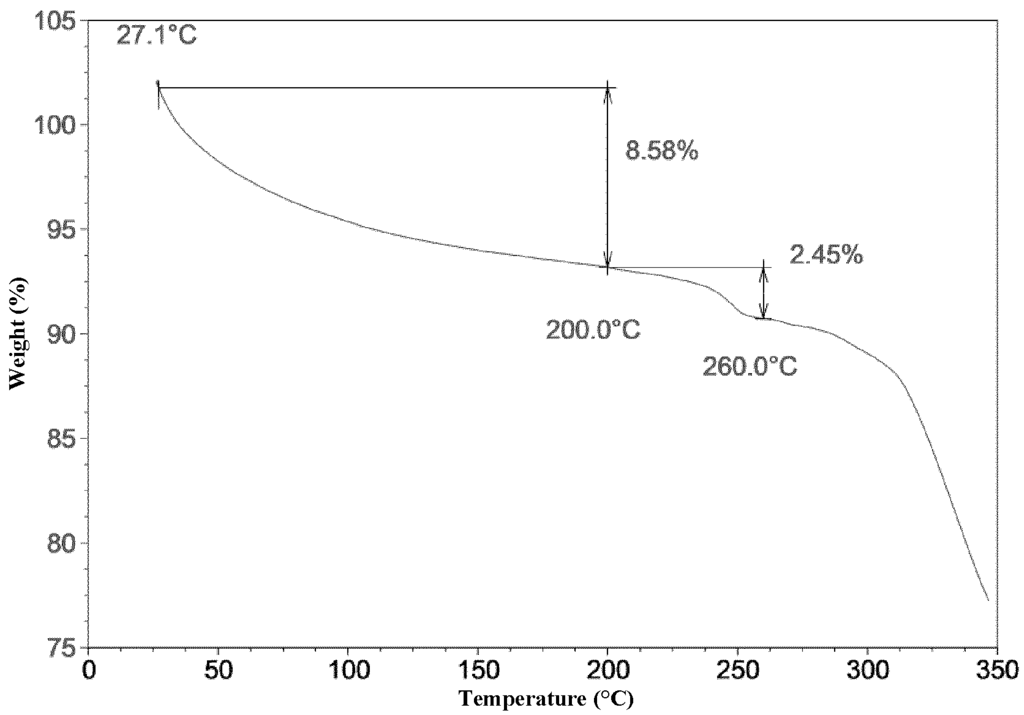

Weight (%)
105
100
95
90
85
80
75
0
50
100
150
200
250
300
350
Temperature (°C)
27.1°C
8.58%
2.45%
200.0°C
260.0°C


FIG. 20

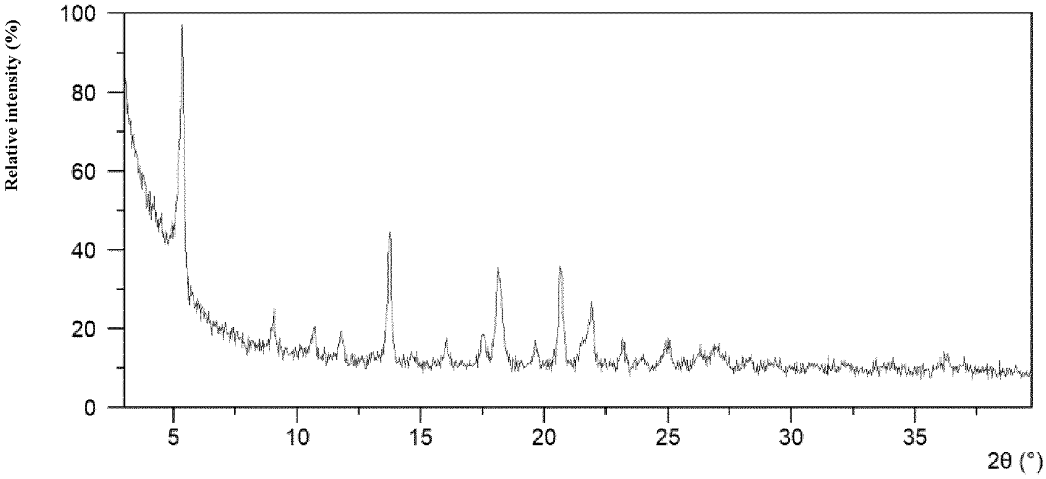


FIG. 21

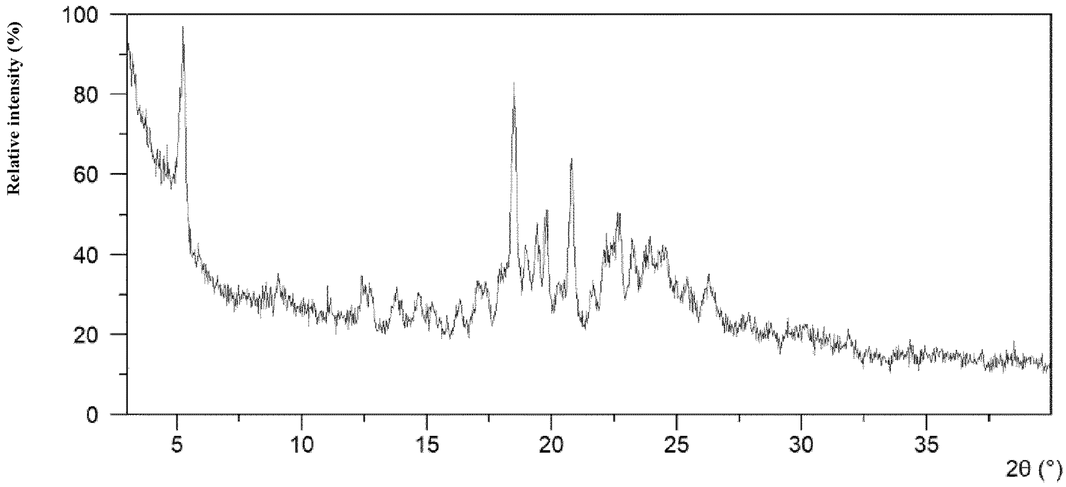


FIG. 22

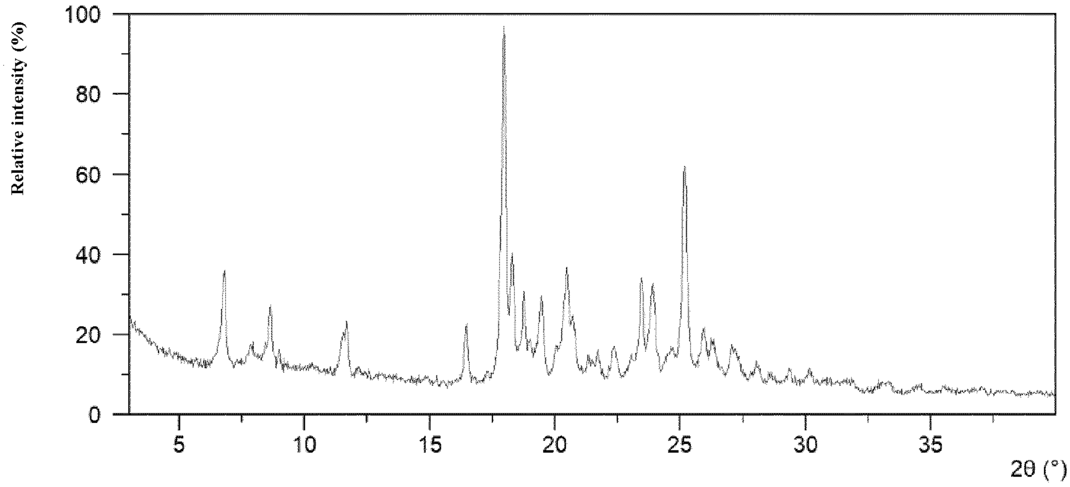


FIG. 23

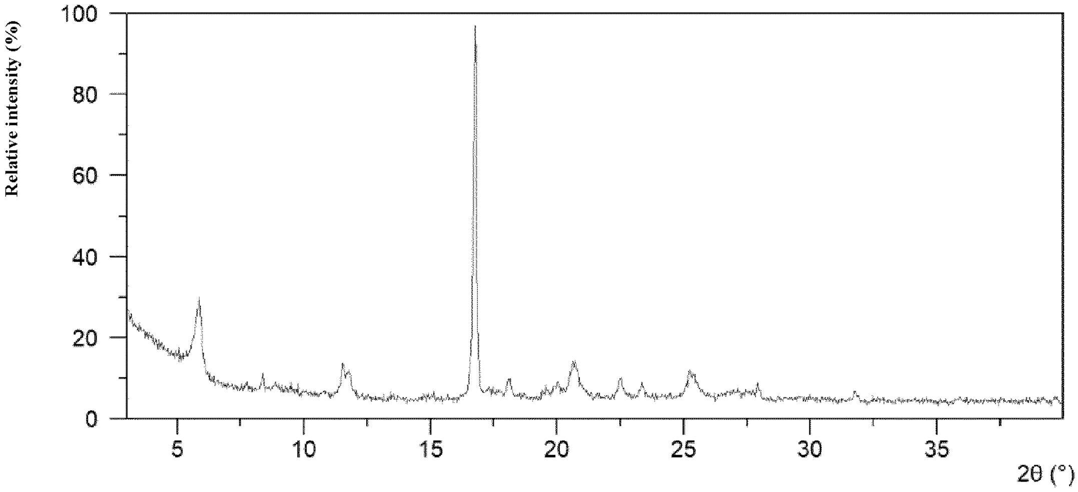


FIG. 24

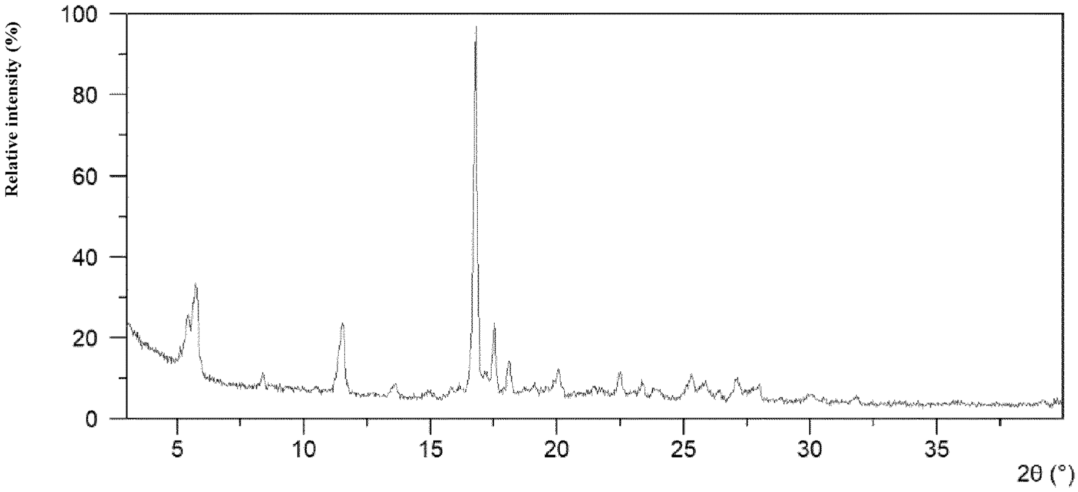


FIG. 25

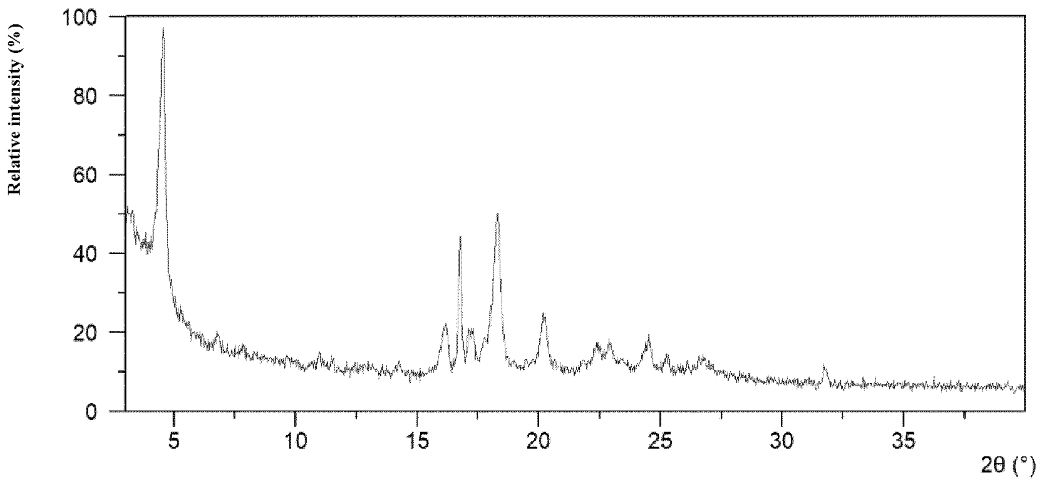


FIG. 26

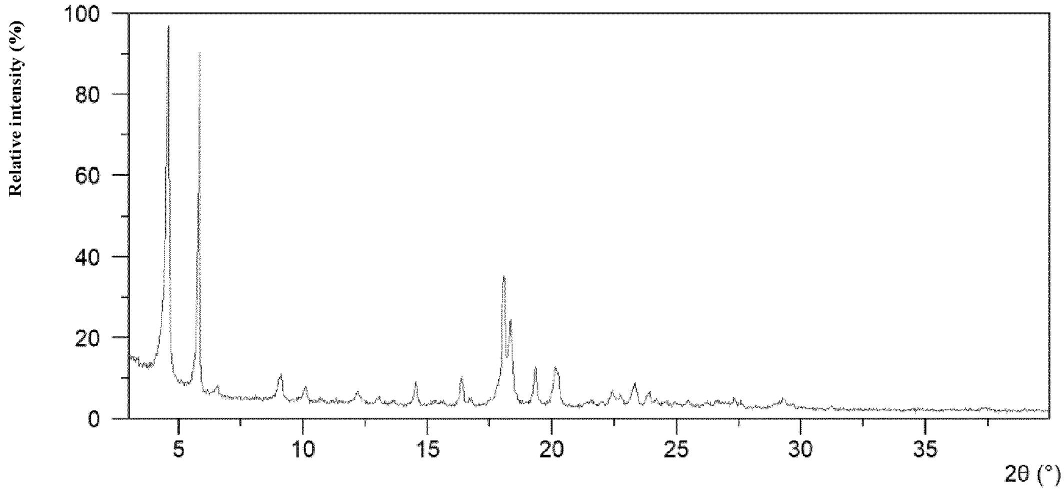


FIG. 27

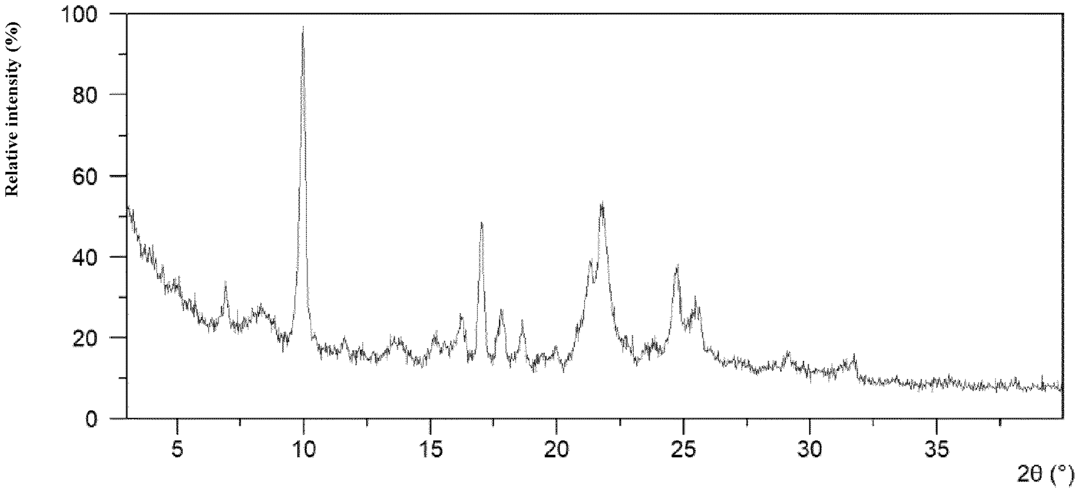


FIG. 28

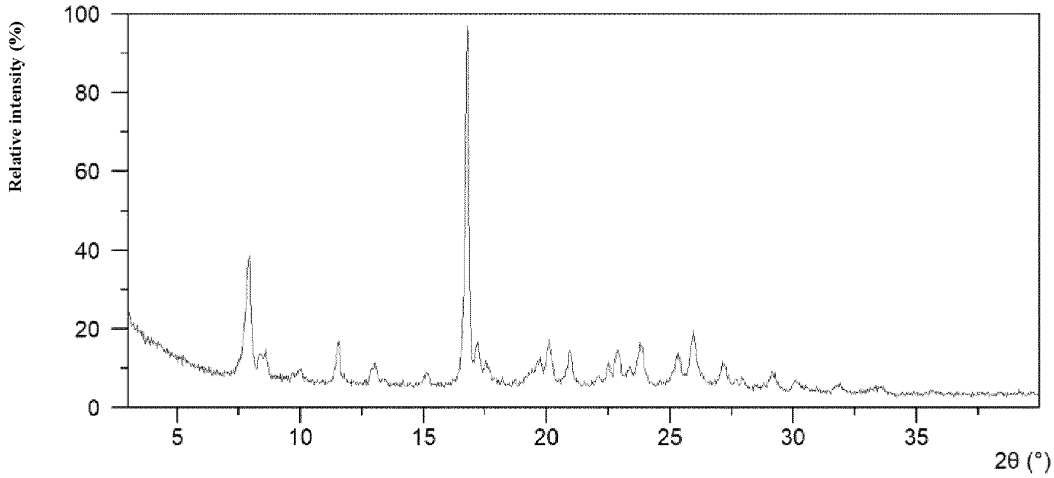


FIG. 29

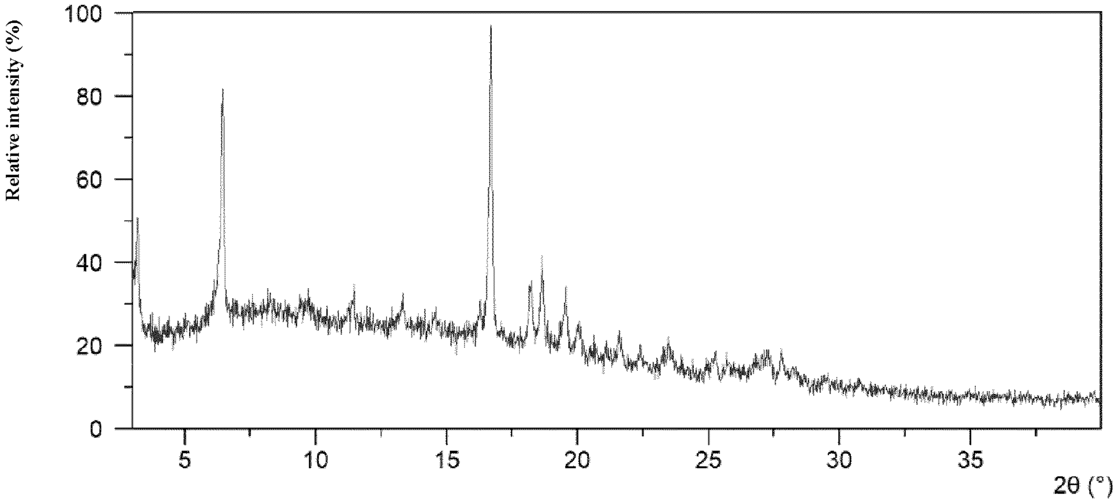


FIG. 30

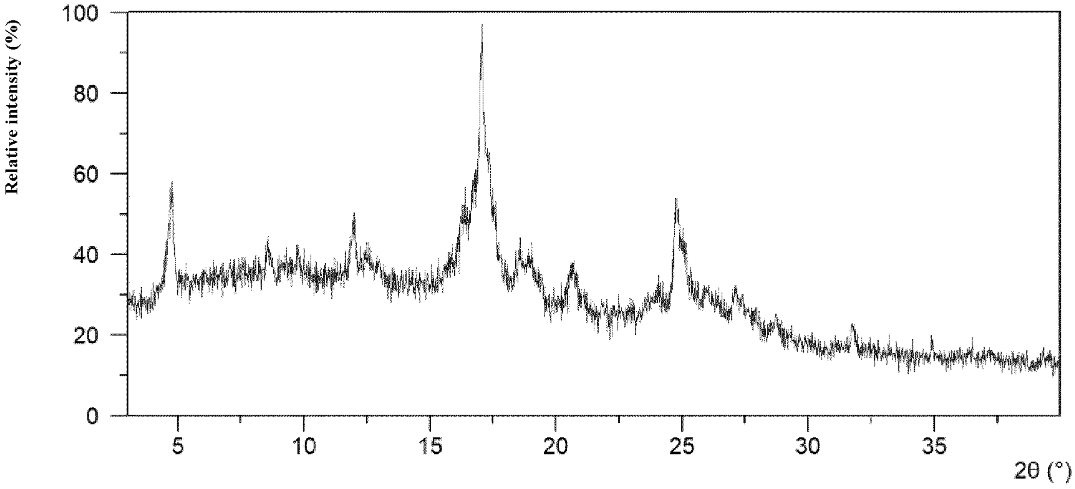


FIG. 31

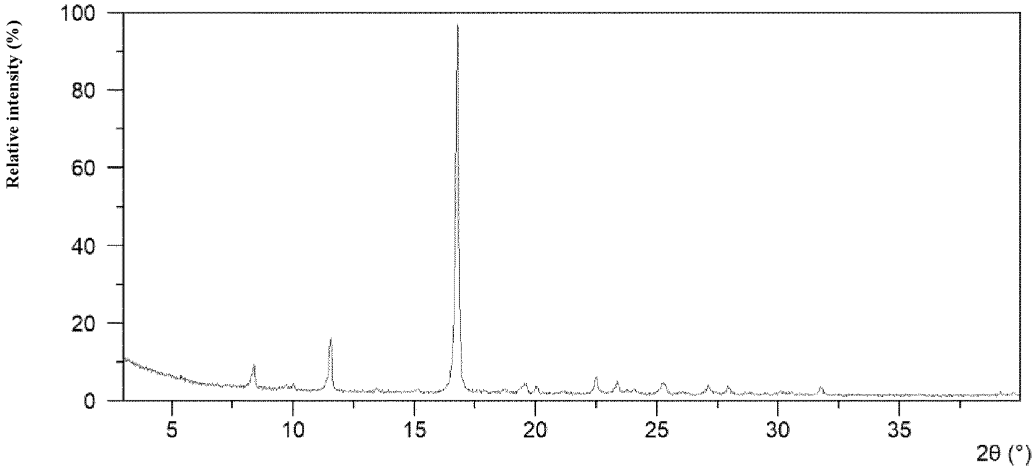


FIG. 32

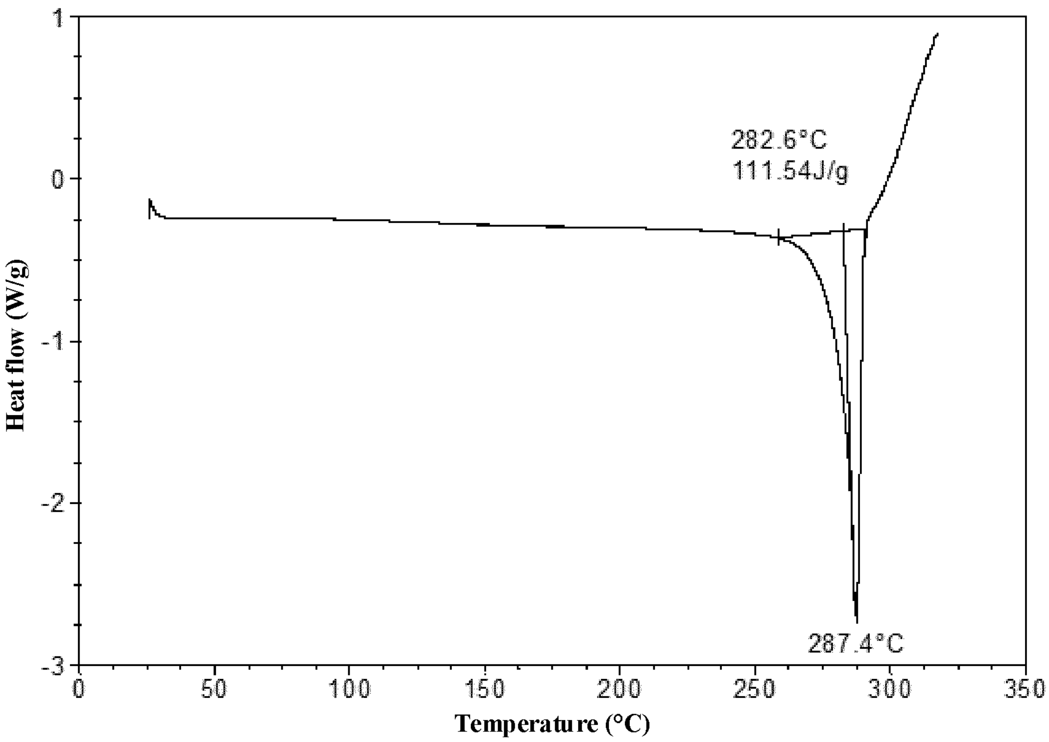

Heat flow (W/g)
1
0
-1
-2
-3
282.6°C
111.54J/g
287.4°C
0
50
100
150
200
250
300
350
Temperature (°C)


FIG. 33

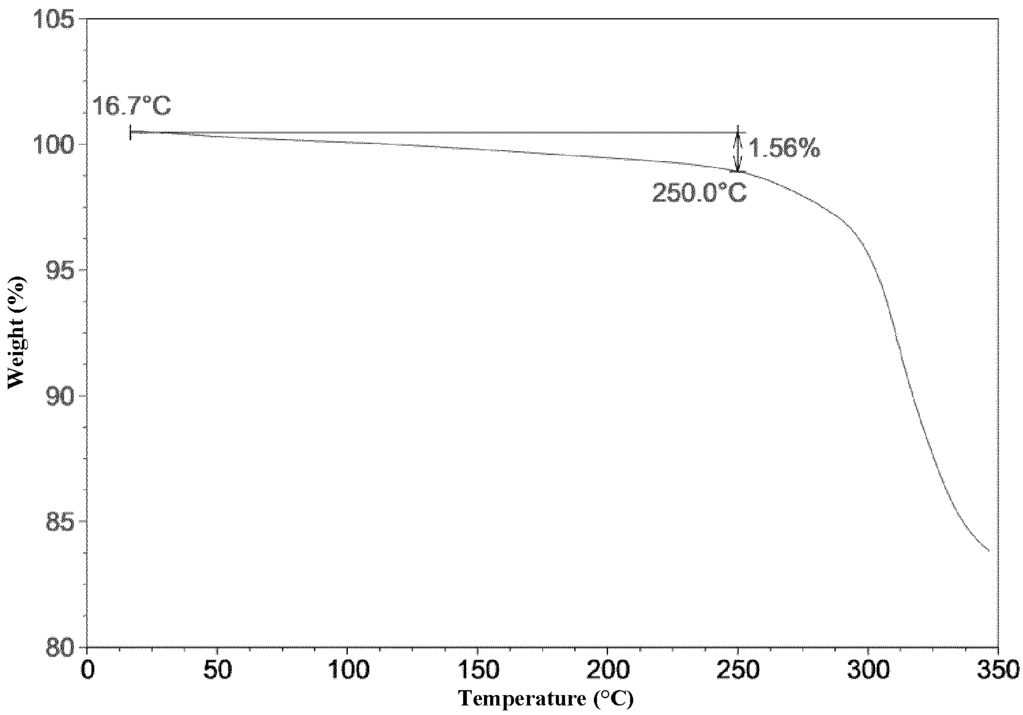

Weight (%)
105
100
95
90
85
80
16.7°C
1.56%
250.0°C
0
50
100
150
200
250
300
350
Temperature (°C)


FIG. 34

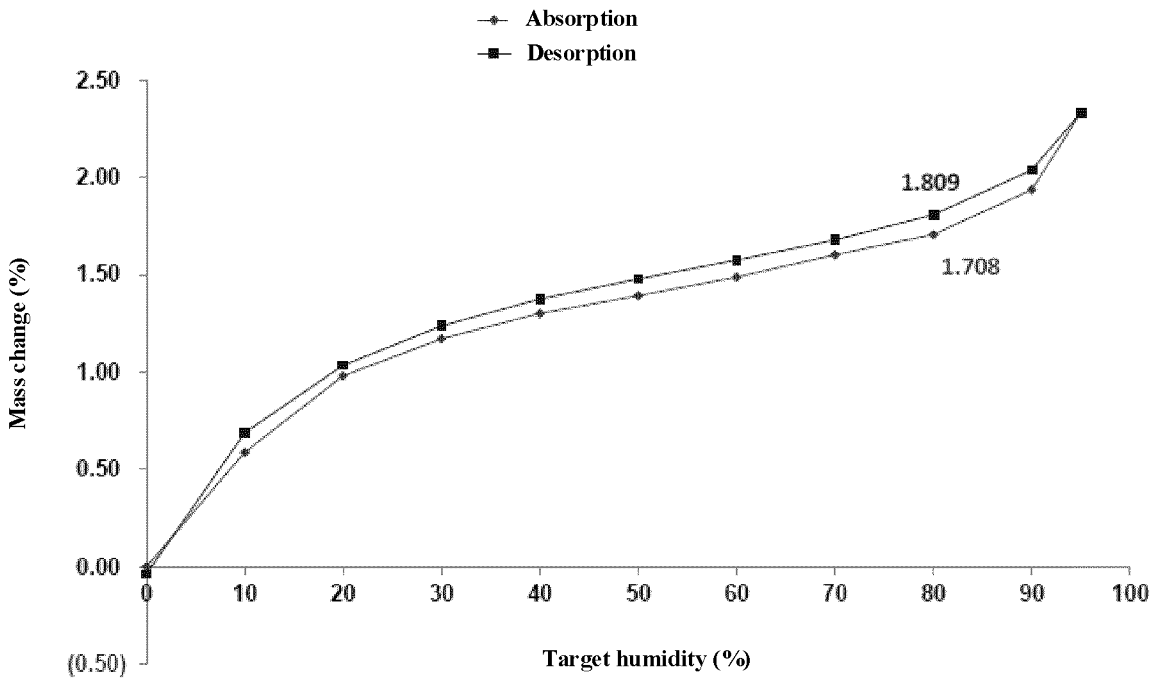
Absorption
Desorption
2.50
2.00
1.50
1.00
0.50
0.00
(0.50)
Mass change (%)
1.809
1.708
0
10
20
30
40
50
60
70
80
90
100
Target humidity (%)

FIG. 35

## INTERNATIONAL SEARCH REPORT

International application No.
**PCT/CN2022/074076**

**A. CLASSIFICATION OF SUBJECT MATTER**

C07D 471/04(2006.01)i; A61P 35/00(2006.01)i; A61K 31/437(2006.01)i

According to International Patent Classification (IPC) or to both national classification and IPC

**B. FIELDS SEARCHED**

Minimum documentation searched (classification system followed by classification symbols)

C07D471/-; A61P35/-; A61K31/-

Documentation searched other than minimum documentation to the extent that such documents are included in the fields searched

Electronic data base consulted during the international search (name of data base and, where practicable, search terms used)

WPI, EPODOC, CNPAT, CNKI, Caplus(STN), Registry(STN): 吡唑, 吡啶, 咪唑, 癌症, 肿瘤, pyrazol+, pyridin+, imidazo+, cancer, tumor, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2021018047 A1 (MEDSHINE DISCOVERY INC.) 04 February 2021 (2021-02-04) claims 15-18 | 1-30 |
| X | WO 2020135878 A1 (MEDSHINE DISCOVERY INC.) 02 July 2020 (2020-07-02) (description page 26 table 1 embodiment 6, page 64, claims 18-21) | 1-30 |
| A | CN 101827844 A (NOVARTIS AG) 08 September 2010 (2010-09-08) claims 1-10 | 1-30 |
| A | WO 2008078091 A1 (ASTEX THERAPEUTICS LIMITED et al.) 03 July 2008 (2008-07-03) claims 1-51 | 1-30 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 April 2022** | **26 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/074076**

| Patent document cited in search report | | | Publication date (day/month/year) | Patent family member(s) | | | Publication date (day/month/year) |
|---|---|---|---|---|---|---|---|
| WO | 2021018047 | A1 | 04 February 2021 | CA | 3145680 | A1 | 04 February 2021 |
| | | | | AU | 2020320997 | A1 | 10 March 2022 |
| WO | 2020135878 | A1 | 02 July 2020 | None | | | |
| CN | 101827844 | A | 08 September 2010 | CL | 2008003056 | A1 | 31 July 2009 |
| | | | | AR | 068877 | A1 | 09 December 2009 |
| | | | | MA | 31766 | B1 | 01 October 2010 |
| | | | | AU | 2008313773 | A1 | 23 April 2009 |
| | | | | CA | 2703037 | A1 | 23 April 2009 |
| | | | | TW | 200922570 | A | 01 June 2009 |
| | | | | PL | 2212323 | T3 | 31 January 2013 |
| | | | | TN | 2010000167 | A1 | 11 November 2011 |
| | | | | BR | PI0817434 | A2 | 16 June 2015 |
| | | | | PE | 20091383 | A1 | 17 October 2009 |
| | | | | JP | 2011500623 | A | 06 January 2011 |
| | | | | IL | 204720 | D0 | 30 November 2010 |
| | | | | ES | 2393430 | T3 | 21 December 2012 |
| | | | | WO | 2009050183 | A2 | 23 April 2009 |
| | | | | EP | 2212323 | A2 | 04 August 2010 |
| | | | | CO | 6270321 | A2 | 20 April 2011 |
| | | | | US | 2010210641 | A1 | 19 August 2010 |
| | | | | EA | 201000615 | A1 | 30 December 2010 |
| | | | | PT | 2212323 | E | 27 November 2012 |
| | | | | MX | 2010004244 | A | 30 April 2010 |
| | | | | ZA | 201002334 | B | 30 March 2011 |
| | | | | KR | 20100076022 | A | 05 July 2010 |
| WO | 2008078091 | A1 | 03 July 2008 | HR | P20150642 | T1 | 14 August 2015 |
| | | | | CN | 101679408 | A | 24 March 2010 |
| | | | | AU | 2007337886 | A1 | 03 July 2008 |
| | | | | CA | 2672172 | A1 | 03 July 2008 |
| | | | | NO | 20092657 | L | 17 September 2009 |
| | | | | AR | 064491 | A1 | 08 April 2009 |
| | | | | MX | 2009006706 | A | 02 July 2009 |
| | | | | JP | 2010513447 | A | 30 April 2010 |
| | | | | US | 2010120761 | A1 | 13 May 2010 |
| | | | | EP | 2114941 | A1 | 11 November 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110106007